# EUROPÄISCHE PATENTSCHRIFT

(11) **EP 3 656 442 B1**
(45) Veröffentlichungstag und Bekanntmachung des Hinweises auf die Patenterteilung: **03.01.2024**
(21) Anmeldenummer: 19209912.5
(22) Anmeldetag: 19.11.2019
(51) Int. Cl.: A61N 2/02

(54) **BEHANDLUNGSSTUHL FÜR DIE MAGNETSTIMULATION VON KÖRPERGEWEBE**
TREATMENT CHAIR FOR THE MAGNETIC STIMULATION OF BODY TISSUE
FAUTEUIL D'EXAMEN DESTINÉ À LA STIMULATION MAGNÉTIQUE DU TISSU CORPOREL

(30) Priorität: 20.11.2018 DE 202018106565 U
(43) Veröffentlichungstag der Anmeldung: 27.05.2020
(73) Patentinhaber: ÖPF Establishment, 9492 Eschen (LI)
(72) Erfinder: FISCHER, Gerhard, 9435 Heerbrugg (CH)
(74) Vertreter: Riebling, Peter

(56) Entgegenhaltungen:
- EP-A2- 2 676 700
- WO-A1-2011/076394
- WO-A1-2011/076395
- WO-A2-2008/146250
- CN-A- 107 137 200
- CN-A- 107 715 302
- DE-A1-102016 116 399
- DE-A1-102017 000 876
- GB-A- 134 640
- JP-A- 2003 070 857
- US-A1- 2017 259 077

## Beschreibung

Die Erfindung betrifft einen Behandlungsstuhl für die Magnetstimulation von Körpergewebe, insbesondere für die transpelvine Magnetstimulation.

Ein Behandlungsstuhl für die transpelvine Magnetstimulation verwendet in der Regel zwei oder mehrere unabhängige Magnetspulen, wobei die eine Magnetspule bevorzugt in der Rückenlehne angeordnet und vorzugsweise entlang der Längsachse der Rückenlehne verschiebbar und feststellbar ist, während eine andere Magnetspule im Bereich der Sitzfläche angeordnet und vorzugsweise in Richtung der Längsachse der Sitzfläche verschiebbar und feststellbar ist.

Die Art und Ausbildung der verwendeten Magnetspulen lässt sich beispielsweise aus der DE 10 2013 014 913 A1 entnehmen.

Ein ähnlicher Stand der Technik ergibt sich auch durch die DE 10 2009 049 145 A1.

Insbesondere die Darstellung des Funktionsprinzips des transpelvinen Behandlungsstuhls wird in der DE 10 2009 049 145 A1 ausführlich erläutert. Auf die dortige Erläuterung wird hingewiesen. Sie soll vollinhaltlich von der Offenbarung der vorliegenden Erfindung umfasst sein.

Als weiterer Stand der Technik wird insbesondere die DE 20 2017 107 602 U1genannt.

Der Einsatz hochintensiver dynamischer Magnetfelder zur Erzeugung von Aktionspotentialen im zentralen aber auch peripheren Nervensystem, ist in der Medizin von wachsendem Interesse. So ist die repetitive transkranielle Magnetstimulation rTMS nicht nur ein anerkanntes Verfahren in der Behandlung therapieresistenter Major Depressions, sondern wirkt auch bei Neuropathien, Fibromyalgie, Migräne, L-Dopa-bedingten Dyskinesien bei Morbus Parkinson oder auch bei Bewegungsstörungen nach Erkrankungen wie MS und ALS. Mit der repetitiven peripheren Magnetstimulation rPMS hat sich inzwischen sich in Form einer transpelvinen Magnetstimulation rTPM eine wirksame Therapieoption in der Behandlung einer Harninkontinenz und von Haltungsschäden entwickelt. Auch hinsichtlich einer Inkontinenz nach Prostatektomie, dem Pelvic-Pain-Syndrom, Post-Partum-Schäden, einer erektilen Dysfunktion sowie der sexuellen Dysfunktion der Frau zeichnen sich positive Wirkungen ab.

Zur Durchführung einer rPMS / rTPM steht auf der Grundlage eigener RCT-Studien ein Medizintechniksystem in Form des erfindungsgemäßen Behandlungsstuhls (PelviCenter) zur Verfügung. Das Pelvicenter knüpft dabei an das grundsätzliche Wirkprinzip einer Magnetstimulation des Beckenbodens an, zu dem in der Datenbank Pubmed (Medline) inzwischen schon eine Vielzahl von klinischen Studien mit teilweise deutlichen oder signifikanten Therapieeffekten in der Behandlung einer Stress-, Drang- und Mischinkontinenz indexiert sind. Auffällig ist dabei, dass sich die jeweiligen Studiendesigns zur Reizkonfiguration weitgehend ähneln und Erkenntnisse aus der Trainingsphysiologie der Skelettmuskulatur nicht immer umgesetzt werden. Da es sich bei der komplexen Schichtarchitektur des Beckenbodens um Skelettmuskeln handelt, erscheint es notwendig, Stimulationseffekte im Hinblick auf den Einsatz optimaler Reizparameter zu quantifizieren. Hierzu hat die Prof. Dr. Fischer AG eine Grundlagenstudie an der Universität der Bundeswehr In München-Neubiberg, Institut für Sportwissenschaften und Sport, durchführen lassen, denen bereits eine Untersuchung der magnetstimulatorischen Reizschwelle (Verschmelzungsfrequenz) eines günstigen Muskeltetanus an der Wadenmuskulatur unter Ultraschallkontrolle vorausgegangen war.

Neben dem Beckenboden werden auch die Hüft-, Gesäß-, Oberschenkel- und sakralen Muskeln intensiv trainiert. Im Folgenden wird das Funktionsprinzip der rPMS am Beispiel einer Magnetstimulationsbehandlung, insbesondere Magnetstimulation bei Belastungs- und Dranginkontinenz, erläutert.

### a. Wirkprinzip

Die repetitive periphere Magnetstimulation (rPMS) basiert auf dem Faradayschen Prinzip ("elektromagnetische Induktion"), bei dem ein veränderliches Magnetfeld (zeitlich und räumlich) in einem leitenden Medium wiederum ein elektrisches Feld proportional zur Änderung des magnetischen Felds erzeugt. Die Stärke des elektrischen Feldes im Gewebe ist abhängig von der absoluten Stärke des erzeugten Magnetfeldes, der Änderungsgeschwindigkeit des Magnetfeldes, dem Abstand der Spule vom zu erregenden Gewebe sowie der elektrischen Leitfähigkeit des Materials. Im Gegensatz zu elektrischen Feldern durchdringen Magnetfelder den Organismus faktisch ungehindert.

Das elektrische Feld wird via Leiterschleife "widerstandslos" auch tief im Perineum und dem darunter liegenden Beckenboden erzeugt, wobei die Feldverteilung einer charakteristischen Glockenform entspricht.

Die Glockenform kommt über die Spulenanordnung zustande und demonstriert die höchste Intensität im Scheitelpunkt und eine zunehmende Abschwächung am Glockenrand bzw. an der Peripherie.

Da sich die Intensität eines Magnetfelds quadratisch im Abstand zur Quelle reduziert, liegt die Eindringtiefe bei maximal 9-10 cm. Die Flussdichte liegt bei mindestens 0,4 Tesla und beträgt in der Spitzenladung (190 µsec) bei 10 000 Tesla/sec.

Im Rhythmus der Frequenzschaltung zwischen 5-50 Hz kommt es im effektiv wirksamen elektrischen Feld zu einer Depolarisation von motorischen Nerven und konsekutiv zu einer starken Kontraktion der quer gestreiften Beckenbodenmuskulatur. Dies ist eine Folge der Freisetzung von Neurotransmittern an der motorischen Endplatte. Gleichzeitig erfolgt ein propriozeptiver Zustrom ins ZNS, der sowohl durch die Afferenzen der stimulierten gemischten Nerven (senso-motorisch), als auch durch die Propriozeptoren (Muskelspindeln, Golgi-Sehnenorgane) erfolgt und im somatosensorischen Cortex ("Repräsentationszentrum") zu entsprechenden Prägungen führt. Als additives Wirkprinzip gilt auch die Stimulation afferenter Fasern der Pudendusnerven und der inhibitorischen Fasern des sympathischen N. hypogastricus, durch die die Detrusormuskeln eine Dämpfung erfährt.

### b. Therapieprozedere

Die Behandlungsparameter leiten sich aus Erfahrungswerten zur Elektrostimulation ab und wurden in die Methodik der meisten Studien übernommen. Diese sehen eine nieder- bzw. höherfrequente Impulsgebung (10 / 50 Hz) über jeweils 10 Minuten vor (insgesamt 20 Minuten), bei einer Gesamttherapiedauer von 6 Wochen und einer Behandlungsfrequenz von zwei Mal pro Woche. Die Therapiedauer orientiert sich dabei an der Maßgabe einer minimalen Wirkungszeit und der Entscheidungswahrscheinlichkeit eines Probanden, vorzeitig aus der Studie auszusteigen.

Insgesamt gibt es deutliche Hinweise, dass die meisten Patienten bis zu diesem Zeitpunkt noch kein optimales Erfolgsplateau erreichen. Da Muskeln nur 36-48 Stunden brauchen, um sich wieder zu regenerieren, ist - bei entsprechender Compliance - eine Erhöhung der Anwendungsfrequenz auf 3 x pro Woche bei gleichzeitiger Reduktion oder Beibehaltung der Behandlungszeit von 6 Wochen, als gleichwertig bzw. sogar überlegen anzusehen.

### c. Modifiziertes Therapieprozedere

Aus den Erfahrungen der Elektrostimulation lassen sich Frequenzbereiche ableiten, die für ein modifiziertes Therapieprozedere der TPM wertvoll sind. So spricht die Stimulation sympathischer Reflexe ("Inhibition") für eine Behandlung der Dranginkontinenz im Bereich von 5-10 Hz; um N. Pudendus und den urethralen Schließmuskel zur Kontraktion zu bringen, jedoch 50 (- 100) Hz. Dieser Frequenzbereich ist damit eher für die Belastungsinkontinenz geeignet.

### d. Langzeitwirkung

Die Stimulationswirkung der rPMS ist nicht nur auf einen kurzen Zeitraum nach einer Behandlungsserie von durchschnittlich 12-18 Anwendungen beschränkt, sondern besteht auch über eine Latenzzeit von 6-12 Monaten fort. So bestand noch 6 Monate nach der Stimulation bei 28-42 % der Patientinnen eine Komplettremission und bei 45-70 % eine anhaltende Verbesserung. Je nach Studie ist sogar mit einer Wirkdauer von bis zu 35 Monaten zu rechnen.

### e. Studienlage

Die rPMS hat ihre Wirksamkeit sowohl studienmäßig als auch in der Praxis unter Beweis gestellt, wobei die Belastungs- und Dranginkontinenz bei OAB sowie die Mischformen zu den Einsatzgebieten zählen. Weitere, noch nicht studienmäßig geklärte Einsatzgebiete sind Inkontinenzen nach radikaler Prostatektomie sowie Stuhlinkontinenzen.

In einer Auswertung von 60 Studien- und Kongressbeiträgen imponieren Erfolgsraten (Symptombefreiung oder deutliche Besserung) zwischen 41 und 78 %, jeweils in Abhängigkeit von Einsatzdauer, Symptomatik, Alter, Vorbehandlung und Begleiterkrankungen. Bemerkenswert sind auch absolute Heilungsraten von bis zu 42 % ("dry"), wobei zu berücksichtigen ist, dass eine Gegenkontrolle mittels Placebo wegen der eindeutigen sensorischen Perzeption systemimmanenter Muskelkontraktionen nur eingeschränkt möglich ist.

Im Hinblick auf allfällige muskuläre Abbauraten nach Stimulationskarenz sollte der rPMS-Erfolg durch ein jährliches Auffrischungstraining auch langfristig gesichert werden.

### f. Kontraindikationen / Nebenwirkungen

Langzeiterfahrungen mit der MRI-Diagnostik (Kernspin) und der Depressionsbehandlung mittels TMS (transkranielle Magnetstimulation), die ein ungleich stärkeres Magnetfeld erzeugen (1-3 Tesla), beweisen, dass die rPMS als ein nebenwirkungsfreies und sicheres Verfahren anzusehen ist. Als Kontraindikationen gelten Schwangerschaft, Insulinpumpen, Herzschrittmacher, Shuntsysteme, Epilepsien und Fremdkörper aus Eisen. Die in der Frakturheilung und Endoprothetik eingesetzten Metalle und Legierungen wie Schrauben, Platten und Implantate (Titan) sind in der Regel nicht ferromagnetisch und damit unbedenklich können aber, wie chirurgischer Stahl, trotz erheblich herabgesetzter Leitfähigkeit zur Erwärmung oder Erhitzung führen. Nebenwirkungen sind nicht bekannt. Zu Behandlungsbeginn wird in Einzelfällen von einem "Muskelkater" im Bereich der Gesäßmuskulatur und des Beckenbodens berichtet.

### g. Einsatzgebiete

- Stress- bzw. Belastungsinkontinenz
- Dranginkontinenz I OAB-Syndrom
- Mixed-Formen
- Stuhlinkontinenz infolge Beckenboden- bzw. Sphinkterschwäche
- Pelvic-Pain-Syndrom I Pelvic-Bladder-Syndrom
- Vaginale Examination für aktives Beckenbodentraining
- Belastungsinkontinenz nach radikaler Prostatektomie I TUR-P

### h. Fazit und Perspektive

Obwohl mit den Standard-Therapieverfahren grundsätzlich zufriedenstellende bis gute Behandlungsergebnisse zu erreichen sind, werden diese vorn Gros der Patienten (60-95 %) nicht angenommen. Daran hat auch die Entwicklung nebenwirkungsärmerer Medikamente oder neuer Operationstechniken, die sowieso nur für einen Bruchteil der Patienten infrage kommt, nichts ändern können.

Gerade im Hinblick auf diese unbefriedigende Akzeptanz stellt die rPMS eine neue, wirksame Behandlungsalternative zu den bisherigen Therapieverfahren dar. Im Gegensatz zu belastenden chirurgischen Eingriffen bzw. medikamentös bedingten, unerwünschten Nebenwirkungen sowie hohen Anforderungen an die Selbstdisziplin im Wege des aktiven Beckenbodentrainings ist sie völlig belastungs- und nebenwirkungsfrei. Weil sich Patienten nicht mehr entkleiden müssen und zudem einen angenehmen sensorischen "Kitzel" passiver Muskelkontraktionen erleben, ist die Compliance sehr hoch.

Die rPMS sollte deshalb nicht nur anhand ihrer Erfolgsquote im Vergleich zu anderen etablierten Therapieverfahren beurteilt werden. Sie bietet vielmehr die Chance, einen Großteil der Inkontinenzkranken, die sich bisher einer Therapie aus Scham und Resignation verschlossen haben, wieder in eine Behandlung zurückzuführen. Die rPMS richtet sich deshalb - neben dem Urologen und Gynäkologen - auch an Allgemeinmediziner und hausärztlich tätige Internisten, deren Nachbetreuungsmöglichkeit sich bisher hauptsächlich in einer anamnestischen Dokumentation erschöpfen musste. Damit gewinnt die rPMS auch eine gesundheitspolitische Dimension, da aus der bisherigen Nichtbehandlung von Millionen von Inkontinenz-Patientinnen sehr viel persönliches Leid und auch ein hoher volkswirtschaftlicher Schaden resultieren.

Die als Stand der Technik genannte DE 20 2017 107 602 U1 zeigt einen Behandlungsstuhl, der sehr komfortabel und bequem ausgestaltet ist, damit die zu behandelnde Person möglichst entspannt die Behandlung genießen kann. Insbesondere umfasst ein solcher Behandlungsstuhl seitliche Armlehnen, die zum einen die Bequemlichkeit des Behandlungsstuhls erhöhen und insbesondere auch Bedienfelder umfassen, die vom Benutzer bequem und leicht erreichbar bedient werden können.

Ein Nachteil dieses Behandlungsstuhls ist, dass der Einstieg aufgrund einer vorhandenen Beinstütze insbesondere für körperlich beeinträchtigte oder behinderte Personen sehr schwer ist. Beispielsweise ist es für einen Rollstuhlfahrer ohne fremde Hilfe nur sehr schwer möglich vom Rollstuhl auf den Behandlungsstuhl umzusteigen.

Die WO 2011/076395 A1 offenbart einen Behandlungsstuhl für die Magnetstimulation von Körpergewebe, bestehend aus einem Grundgestell mit einer Sitzfläche und einer Rückenlehne, sowie zwei Seitenteilen mit Armlehnen, wobei in der Sitzfläche und/oder der Rückenlehne mindestens eine Magnetspule angeordnet ist, wobei zumindest eines der beiden Seitenteile verschiebbar am Grundgestell angeordnet ist, derart, dass ein seitlicher Zugang zur Sitzfläche des Behandlungsstuhls freigegeben wird.

Die WO 2011/076394 A1 offenbart einen ähnlichen Behandlungsstuhl wie WO 2011/076395 A1 wobei zumindest eines der beiden Seitenteile klappbar am Grundgestell angeordnet ist. Die GB 134 640 A offenbart ebenfalls einen Behandlungsstuhl mit schwenkbarem Seitenteil.

Die WO 2008/146250 A2 offenbart einen Behandlungsstuhl zur Magnetstimulation, der zwei Elektroden in Form von Haltestangen aufweist, die zur Pulsmessung dienen.

Der Erfindung liegt die Aufgabe zugrunde, einen Behandlungsstuhl der eingangsgenannten Art so weiterzubilden, dass der Einstieg auf den Behandlungsstuhl wesentlich erleichtert wird, so dass insbesondere auch körperlich beeinträchtigte oder behinderte Personen, beispielsweise Rollstuhlfahrer, selbständig und bequem auf den Behandlungsstuhl einsteigen können, und die Effektivität der Magnetstimulation zu verbessern.

Zur Lösung der gestellten Aufgabe ist die Erfindung durch die technische Lehre des Patentanspruches 1 gekennzeichnet.

Der Behandlungsstuhl für die transpelvine Magnetstimulation (TPM / rPMS) besteht aus einem Grundgestell mit einer Sitzfläche und einer Rückenlehne, sowie zwei Seitenteilen mit Armlehnen, wobei in der Sitzfläche und/oder der Rückenlehne mindestens eine Magnetspule angeordnet ist. Die Magnetspulen sind in Längsrichtung und/oder Querrichtung der jeweiligen Sitzfläche und/oder Rückenlehne verschiebbar angeordnet.

Erfindungsgemäß ist zumindest eines der beiden Seitenteile in Längsrichtung der Sitzfläche derart verschiebbar, dass die Sitzfläche seitlich frei zugänglich ist und ein Seiteneinstig auf die Sitzfläche leicht möglich ist. An den Seitenteilen oder Armauflagen sind Haltegriffe zum Ziehen durch den Benutzer angeordnet, die zur Messung einer Steigerung der Muskelkraft aufgrund der Magnetstimulation ausgebildet sind. Weiter ist ein Bedienpanel mit einem Display zur grafischen Anzeige der Stellung der Magnetspulen und der Leistungssteigerung über die Haltegriffe ausgebildet.

Somit ist ein komfortabler Einstieg der zu behandelnden Person möglich. Insbesondere körperlich beeinträchtigten oder behinderten Personen wird der Einstieg auf den Behandlungsstuhl wesentlich erleichtert, da sie nun direkt von der Seite Zugang zur Sitzfläche haben.

Nach dem Einsteigen der zu behandelnden Person auf den Behandlungsstuhl kann das verschiebbare Seitenteil wieder in seine Ausgangslage zurück verschoben werden, so dass beide Armlehnen wie gewohnt zur Verfügung stehen und ein komfortables Sitzen während der Behandlung unterstützen.

In einer bevorzugten Ausgestaltung der Erfindung ist das verschiebbare Seitenteil des Behandlungsstuhls nach hinten in Richtung der Rückenlehne verschiebbar, wodurch die Sitzfläche seitlich freigegeben wird und der Benutzer seitlich auf die Sitzfläche einsteigen kann.

Das verschiebbare Seitenteil ist am Grundgestell des Behandlungsstuhls angeordnet und vorzugsweise in einer Führungsschiene entlang des Grundgestells verschiebbar gelagert. Das Verschieben des Seitenteils kann manuell oder automatisch mittels Motorantrieb erfolgen.

Gemäß einer anderen Ausgestaltung der Erfindung kann es vorgesehen sein, dass das Seitenteil abklappbar am Grundgestell angeordnet ist. Hierbei wird es bevorzugt, wenn dass das Seitenteil um eine am hinteren Teil des Grundgestells angeordnet vertikale Schwenkachse nach hinten verschwenkbar ist und dadurch die Sitzfläche seitlich freigibt, so dass der Benutzer sich von der Seite auf die Sitzfläche setzen kann. Die Schwenkachse kann durch ein Scharnier oder Zapfenlagerung gebildet sein. Das Verschwenken des Seitenteils kann manuell oder automatisch mittels Motorantrieb erfolgen.

Um das Verschieben oder das Schwenken des Seitenteils zu erleichtern und die Führungsschiene bzw. das Scharnier des Seitenteils teilweise zu entlasten kann es vorgesehen sein, dass an der dem Boden zugewandten Fläche des Seitenteils mindestens eine Stützrolle angeordnet ist, die beim Verschieben oder Schwenken des Seitenteils auf dem Boden abrollt.

In einer vorteilhaften Ausgestaltung der Erfindung umfasst der Behandlungsstuhl eine an die Sitzfläche anschließende und gegenüber der Sitzfläche verschwenkbare Beinstütze. Im Bereich der Beinstütze eine kann eine weitere Magnetspule angeordnet sein.

Gerade für Personen, die sich im Alltag, wie beispielsweise Rollstuhlfahrer, wenig bewegen (können), ist der Behandlungsstuhl "Pelvicenter" für die Muskelstimulation äußerst gut geeignet. Rollstuhlfahrer (Querschnittsgelähmte) berichten in einem Feldversuch, dass sich ihre "toten" Muskeln durch die Magnetstimulation wieder kontrahieren.

Der Patientenkreis der Rollstuhlfahrer setzt sich in der Regel aus Querschnittsgelähmten (Paraplegiker), Schlaganfall- sowie Multiple-Sklerose-Patienten zusammen. Querschnittsgelähmte können dabei an einer inkompletten motorischen (Parese mit Restmotorik und Restsensibilität) oder an einer kompletten motorischen Lähmung (Plegie mit Verlust der Empfindungen, wie Druck, Schmerz und Berührungen) leiden. Bei den Patienten, die einen Rollstuhl bedienen können, handelt es sich in der Regel um spinale Läsionen im Brust-, Lenden oder Sakralbereich (Paraplegie). Hinzu kommen in der Regel Blasenstörungen sowie bei bis zu 80 % der Männer eine erektile Dysfunktion, wobei der Ausprägungsgrad von der unterbrochenen Rückenmarksebene abhängig ist (Courtois FJ, Charvier KF, Leriche A et al. Sexual function in spinal cord injury men. I. Assessing sexual capability. Paraplegia 1993;31:771-784). Allerdings bleibt bei Verletzungen oberhalb L3 der autonome Spinalreflex einer Erektion erhalten, so dass hier durch Manipulationsreize meist eine Erektion auslösbar ist (Löchner-Ernst D. Mit Sildenafil Koitus trotz Querschnittslähmung. Ärzte Zeitung. 18.01.2005).

Nach einem ischämischen Insult oder einer intracerebralen Blutung gehört die Lähmung mit 80 % zum häufigsten Erscheinungsbild eines Schlaganfalls. Je nach Region und Ausmaß, handelt es sich meist um eine Hemiplegie, also eine Lähmung der kontralateralen Körperhälfte mit verminderter Kraftentfaltung der Beinmuskulatur oder z.B. der kleinen Glutealmuskeln, mit einer entsprechenden Gangunsicherheit oder einem Gangverlust (Minussymptomatik nach Fries: Fries W, Freivogel S, Beck, B. Rehabilitation von Störungen der Willkürmotorik. In: Frommelt, P. & Grötzbach, H. (Hrsg.). Neurorehabilitation. Berlin: Blackwell. 1999). Durch Enthemmung der vom Hirnstamm absteigenden Bahnen entwickelt sich daraus eine spastische Lähmung mit Tonuserhöhung der Muskulatur (Plussymptomatik nach Fries), gesteigerten Muskeleigenreflexen und einem Verlust der Feinmotorik (O'Dwyer NJ, Ada L, Neilson PD. Spasticity and muscle contracture following stroke. Brain. 1996;119: 1737-1749). Hinzu kommen meist somatosensorische Störungen (Carey LM. Somatosensory loss after stroke. Critic Rev Phys Rehabil Med. 1995; 7: 51-91) mit Störung der Oberflächen- oder auch der Tiefensensibilität (Neglect). Je nach Ausprägung und geschädigtem Hirnbereich leidet die Mehrzahl der männlichen Patienten an einer erektilen Dysfunktion (Koehn J, Crodel C, Deutsch M. Erectile dysfunction (ED) after ischemic stroke: association between prevalence and site of lesion. Clin Auton Res. 2015; 25(6): 357-365).

Bei der Multiplen Sklerose (MS) kommt es bei der progredienten Verlaufsform letztendlich zu einem völligen Funktionsverlust der motorischen Nerven, der einer Querschnittslähmung entspricht.

### rPMS / Schlaganfall / Rehabiliation Paresen und Spastiken

Spastiken prägen nicht nur das Erscheinungsbild nach Querschnittslähmung, Schlaganfall oder MS, sondern sind auch allgemeiner Ausdruck oder Symptom der Schädigung kortikospinaler Nervenbahnen bei Ischämien, Blutungen, Fehlbildungen, Entzündungen, Tumoren und sonstigen neurogenerativen oder metabolischen Erkrankungen. Sie sind letztendlich ein komplexes Geschehen, da sich Schädigungen der pyramidalen und extrapyramidalen Nervenbahnen auf verschiedene Ebenen bzw. unterschiedlichen Hirn- und Rückenmarksareale erstrecken können (Zäch G, Koch H. Hrsg.. Paraplegie. Ganzheitliche Rehabilitation. Basel, Karger. 2006: 261-272). Aus Untersuchungen einer Paraplegie, also einem kompletten oder inkompletten Verlust von Sensibilität und Beweglichkeit der unteren Extremität nach Querschnittslähmung oder Poliomyelitis, gibt es Hinweise, dass eine Spastik nicht allein auf neuronalen Veränderungen im Rückenmark beruht, sondern bei der auch sekundäre Veränderungen der Muskelfasern eine Rolle spielen (Dietz V. Syndrom der spastischen Parese. Akt Neurol 2001; 28(2): 49-52). Zur Prävalenz gibt es keine genauen Zahlen. Zusammen mit den etwa 50 000 infantilen Cerebralparesen, wird sie in Deutschland auf 250 000 - 300 000 geschätzt (Reichel G, Wissel J. (Hrsg.). Therapieleitfaden Spastik -Dystonien. Uni-Med Science Verlag. 6. neubearb. Auflage 2017).

Zur Therapie der Spastik gibt es zwar unterschiedliche Therapieansätze wie z.B. eine intrathekale Infusion mit dem Muskelrelaxantium Baclofen, dem lokalen Einsatz von Botulinumtoxin oder physio- und ergotherapeutischen Maßnahmen. Insgesamt werden die Therapieergebnisse aber als eher unbefriedigend betrachtet (Pandey K. Spasticity treatment & management. Medscape Mar 01, 2018 / Rekand T. Clinical assessment and management of spasticity: a review. Acta Neurol Scand: 2010: 122 (Suppl. 190): 62-66). Dies hat hinsichtlich physiotherapeutischer Interventionen wohl auch damit zu tun, dass der propriozeptive Zustrom ins ZNS nur über passiv ausgeführte Bewegungen zustande kommt. Das ist vor allem beim Schlaganfall problematisch, weil grundsätzliche Reorganisationsprozesse mit einer kortikalen Vergrößerung der kontralateralen Extremitätenpräsenz verbunden sind. Dieser "erlernte Nichtgebrauch", z.B. der nicht mehr benutzten Hand, führt zu einem verringerten propriozeptiven Zustrom und damit zu ungewollten neuroplastischen Veränderungen (Taub, E. Somatosensory deafferentation research with monkeys: Implications for rehabilitation medicine. In Ince LP (Ed.), Behavioral Psychology in Rehabilitation Medicine: Clinical Applications (pp. 371-401). New York. 1980. Williams & Wilkins).

Abgeleitet aus der funktionellen Elektrostimulation (und sonstigen passiven rhythmischen Bewegungen oder einer Muskelvibration), unter der eine Verbesserung der motorischen Funktionen und Reduktion des spastischen Muskeltonus berichtet wird (Hummelsheim H, Maier-Loth ML, Eickhof C. The functional value of electric muscle stimulation for the rehabilitation of the hand in stroke patients. Scand J Rehabil Med. 1997: 29: 3-10 / Ng SSM, Hui-Chan CWY.

Transcutaneous electrical stimulation on acupoints combined with task-related training to improve motor function and walking performance in an individual 7 years poststroke: a case study. J Neurol Phys Ther. 2010; 34: 208-213) und über den Umweg der repetitiven transkraniellen Magnetstimulation (rTMS), zieht die relativ junge Fachdisziplin der repetitiven peripheren Magnetstimulation (rPMS) immer mehr Aufmerksamkeit auf sich. Denn während es der funktionellen Elektrostimulation (gewebs)widerstands- und schmerzbedingt an Tiefenwirkung mangelt ("Reizung der Nozizeptoren") und die rTMS die Neuronen der Hirnrinde frequenzabhängig hemmt oder aktiviert, ist mit der rPMS sowohl eine sakrale Wurzelreizung, als auch die Stimulation peripherer motorischer Nerven und damit Muskeln völlig schmerzfrei durchführbar (Struppler A, Angerer B, Gündisch C, Havel P: Modulatory effect of repetitive peripheral magnetic stimulation on skeletal muscle tone in healthy subjects: stabilization of the elbow joint. Exper Brain Res. 2004 15: 59-66). Dabei entsprechen die damit erzeugten afferenten Signale weitgehend denen der verloren gegangenen physiologischen aktiven Willkürbewegungen. Voraussetzung hierfür ist immer die Unversehrtheit der peripheren Nervenbahnen, d.h. es sollten myelinisierte Nervenfasern vorhanden sein.

Bei Schlaganfallpatienten führt eine rPMS zu einer Erhöhung der neuronalen Aktivität des parieto-frontalen Netzwerkes, zur Senkung des spastischen Muskeltonus und auch Verbesserung der Willkürmotorik und Sensorik der zentral gelähmten Bereiche (Krause P, Edrich T, Straube A. Lumbar repetitive magnetic stimulation reduces spastic tone increase of the lower limbs. Spinal Cord. 2004; 42: 67-72 / Struppler A, Jacob C, Müller-Barna P et al. Eine neue Methode zur Frührehabilitation zentral- zentralbedingter Lähmungen von Arm und Hand mittels peripherer Magnet-stimulation. Zeitschrift Elektroenzephalo Elektromyo Verwand Gebiete. 1996; 27: 151-157 / Struppler A, Havel P, Muller-Barna P et al. A new method for rehabilitation of central palsy of arm and hand by peripheral magnetic stimulation. Neurol Rehabil 1997; 3: 145-158 / Struppler A, Angerer B, Havel P. Modulation of sensorimotor performances and cognition abilities induced by RPMS: clinical and experimental investigations. Suppl Clin Neurophysiol 2003; 56: 358-367 / Krause P, Edrich T et al., Anhaltender Einfluss repetitiver Magnet-stimulation der unteren Extremität auf die spastische Tonuserhöhung. Suppl Akt Neurol 2000; 27: S179 / Krause P, Straube A. Repetitive magnetic and functional electrical stimulation reducespastic tone increase in patients with spinal cord injury. Suppl Clin Neurophysiol2003; 56: 220-225 / Krause P, Straube A. Reduction of spastic tone increase induced by peripheral repetitive magnetic stimulation is frequency-independent. Neuro Rehabilitation 2005; 20:63-65 / Struppler A, Binkofski F, Angerer B et al. A fronto-parietal network is mediating improvement of motor function related to repetitive peripheral magnetic stimulation: A PET-H2O15 study. Neurolmage. 2007; 36: 174-186 / Havel P, Struppler A. First steps in functional magnetic stimulation (FMS)- movements of forearm and fingers induced by closed-loop controlled FMS. Acta Physiol Pharmacol Bulgarica. 2001; 26:185-188), wobei sich das auch auf die Frührehabilitation in den ersten Wochen nach Schlaganfall bezieht (Premoselli L. Periphere Magnetstimulation zur Frührehabilitation zentral-bedingter Lähmungen von Arm und Hand in den ersten Wochen nach Schlaganfall. Dissertation. Neurophysiologische Abteilung für Neurophysiologie. Universität Ulm. 2012) Eine rPMS wirkt aber auch bei der inkompletten Querschnittslähmung sowie allen Verlaufsformen der MS (Krafttraining). In Rehabilitationseinrichtungen wird aus Gründen einer Mobilitätsverbesserung vor allem die Arbeit an der Beinmotorik priorisiert, da sich Paresen des Beines besser regenerieren lassen als am Arm (Hesse S, Werner C, Bardeleben A: Der schwer betroffene Arm ohne distale Willküraktivität - ein "Sorgenkind" der Rehabilitation nach Schlaganfall ?! Neurol Rehabil. 2004; 10: 120-126).

Infolge der direkten afferenten Signale scheint es sogar möglich zu sein, mit niederschwelligen rPMS-Reizen, die als solche nicht ausreichen, in den Motorneuronen Aktionspotentiale zu erzeugen, die sensiblen Anteile des gemischten Nervenbündels zu stimulieren und dadurch einen propriozeptiven Zustrom zu entfachen. Dies ließ sich nachweisen, indem mittels niederschwelligen Stimulationsreizen sog. SEPs (Somatosensorisch Evozierte Potentiale) im ZNS entstanden (Kunesch E, Knecht S, Classen J et al. Somatosensory evoked potentials (SEPs) elicited by magnetic nerve stimulation. Electroenceph Clin Neurophysiol. 1993; 88(6): 459-467). Auf jeden Fall ist die durch eine rPMS erzeugte Propriozeption erheblich stärker einzuschätzen, als wie sie durch passive Bewegung entstehen kann (Struppler A, Havel P, Muller-Barna P et al. A new method for rehabilitation of central palsy of arm and hand by peripheral magnetic stimulation. Neurol Rehabil 1997; 3: 145-158 / Struppler A, Angerer B, Gündisch C et al. Modulatory effect of repetitive peripheral magnetic stimulation on skeletal muscle tone in healthy subjects: stabilization of the elbow joint. Experimental Brain Research; 2004; 15: 59-66). Dies kommt dem erheblichen Potential des ZNS zugute, sich durch Umorganisation an veränderte Bedingungen anzupassen (Rossini PM, Pauri F. Neuromagnetic integrated methods tracking human brain mechanisms of sensorimotor areas "plastic" reorganisation, Brain Res. 2000; 33: 131-154).

### Sonstige Effekte

Dünne Fasern der Gruppe III (A-Delta) und IV (C) (Klassifikation der afferenten Nervenfasern nach Reflexstudein von Lloyd / Hunt / In Klammer: Klassifikation nach Summenaktionspotentialmessungen von Erlanger / Gasser) sowie Mechano- und Nozizeptoren der Haut werden nicht mitaktiviert.

### Wirkmodell

rPMS-Effekte entstehen via motorischer Nervenreizung nicht nur in Form einer direkten Muskelkontraktion, sondern auch durch eine Stimulation sensorischer Anteile ("afferent") der dort vorhandenen gemischten Muskelnerven ("motorische und sensorische Endaufzweigungen"). Gleichzeitig entstehen im Falle eines Schlagan-falls durch die bewegungsabhängigen Propriozeptoren (Mechanorezpetoren) in Form von Muskelspindeln ("Längenmessung der Muskelfasern" / Ia- und II-Fasern) sowie von Golgi-Sehnenorgane ("Spannungsmessung" / Ib-Fasern) orthodrome Muskelkontraktions-Signale ins ZNS. Deshalb erfolgt ein propriozeptiver Zustrom immer doppelt: Einmal direkt über den sensorisch-afferente Reizweiterleitung ans ZNS, und dann nochmals indirekt durch die Mechanorezptoren infolge Muskelkontraktion (Struppler A, Angerer B, Havel P: Modulation of Sensorimotor Performances and Cognition Abilities induced by RPMS: Clinical and Experimental Investigations. In Paulus W, Tergau F, Nitsche MA, Rothwell JC, Ziemann U, Hallett M (Hrsg) Supplements to Clinical Neurophysiology Volume 56 - Transcranial Magnetic Stimulation and Transcranial Direct Current Stimulation, 1. Aufl, Elsevier, Amsterdam. 2003: 358-367). Das hat wiederum eine antidrome Gegenbewegung zur Folge, die eine Stimulation der Alpha-Motoneurone bedeutet (Struppler A, Havel P, Muller-Barna P et al. A new method for rehabilitation of central palsy of arm and hand by peripheral magnetic stimulation. Neurol Rehabil 1997; 3: 145-158). Dies entspricht dem physiologischen Prinzip passiver rhythmischer Bewegungen oder auch einer Elektrostimulation peripherer Nerven, die zu einer gesteigerten Erregbarkeit der kortikospinalen Bahnen zur damit stimulierten Muskulatur führen (Lewis GN, Byblow WD. Modulations in corticomotor excitability during passive upper-limb movement: is there a cortical influence? Brain Res. 2002; 943: 263-75 / Ridding MC, Brouwer B, Miles TS et al. Changes in muscle response to stimulation of the motor cortex induced by peripheral nerve stimulation in human subjects. Exp Brain Res. 113 (2000): 135-143). Insgesamt bessert ein rPMS-bedingter propriozeptive Zustrom den "erlernten Nichtgebrauch" der nicht mehr benutzten Extremität und stimuliert gleichzeitig die Sensomotorik bzw. nicht beschädigtes Hirngewebe zur Übernahme verlustig gegangener Bewegungsfunktionen. Dies geschieht nach dem Prinzip wiederholten aktiven Übens (motorisch und sensorisch), das zur Aktivierung "stiller" und gehemmter synaptischer Verbindungen führt und so die kortikale Repräsentation der entsprechenden Hirnrindenareale (Gyrus prae- und postcentralis des somatosensorischen Cortex) für die Extremitäten erweitert.

Die eigentliche muskuläre Reaktion entspricht dem Modell kontraktionsbedingter Mikroverletzungen, der eine Entzündung mit katabolen Abbauvorgängen folgt (Evans, WJ. Effects of exercise on senescent muscle. Clin Orthopaed Rel Res. 2002; 403(Suppl.): S211-S220 / Hill M, Goldspink G. Expression and splicing of the insulinlike growth factor gene in rodent muscle is associated with muscle satellite (stem) cell activation following local tissue damage. J Physiol.2003; 549: 409-418)- Diese Muskeldegeneration wiederum stimuliert Reparaturprozesse, welche durch "myogene Stammzellen" (Satellitenzellen) zustande kommen (Morgan JE, Partridge TA. Muscle satellite cells. Int J Biochem Cell Biol. 2003; 35(8): 1151-1156). Auch Mikroverletzungen des muskulären Bindegewebes (Endo-, Peri- und Epimysium) erzeugen einen starken Trainings- bzw. Wachstumseffekt. Andererseits wird durch ein Widerstandstraining und damit auch einer rPMS immer auch die Synthese des Wachstumshormons IGF-1 stimuliert (Brahm H, Pehl-Aulin K, Saltin B et al. Net fluxes over working thigh of hormones, growth factors and biomarkers of bone metabolism during short lasting dynamic exercise. Calc Tiss Int. 1997;60: 175-180), das starke anabole Eigenschaften besitzt (McKay BR, O'Reilly CE, Phillips SM et al. Co-expression of IGF-1 family members with myogenic regulatory factors following acute damaging musclelengthening contractions in humans. J Physiol. 2008; 15: 5549-5560). Außerdem ist bei regelmäßigem Training auch eine Verstärkung die Androgen-Rezeptorendichte zu erwarten (Inoue K, Yamasaki S, Fushiki T et al. Rapid increase in the number of androgen receptors following electrical stimulation of the rat muscle. Eur J Appl Physiol. 1993; 66: 134-140), nachdem ein Widerstandstraining oder starke Muskelkontraktionen nicht nur IGF-1, sondern auch Testosteron aktivieren, was die Protein-Syntheserate erhöht und den Proteinabbau verhindert (Buresh R, Berg K, French J. The effect of resistive exercise rest interval on hormonal response, strength, and hypertrophy with training. J Strength Cond Res. 2009; 23: 62-71)

Zu den spastikvermindernden Effekten existiert noch kein schlüssiges Modell. Es kommen hier bahnende und hemmende Mechanismen in Frage (Will D. Einfluss der repetitiven peripheren Magnetstimulation (RPMS) auf die Gelenkstabilisierung - Versuch eine Modulation auf kortikaler Ebene mittels transkranieller magnetischer Doppelstimulation nachzuweisen. Dissertation an der TU München. Lehrstuhl für Neurologie und klinische Neurophysiologie. 2007).

### Studien Schlaganfall / Reizkonfiguration

Bezüglich des kortikalen Veränderungspotentials nach peripherer rPMS hängt die Effektstärke nicht nur von Stimulationsparametern wie Frequenz oder der Amplitude ab (Chipchase LS, Schabrun SM, Hodges PW. Peripheral electrical stimulation to induce cortical plasticity: A systematic review of stimulus parameters. Clin. Neurophysiol. 2011; 122:456-463), sondern selbstverständlich auch von der Länge und Anzahl der Anwendungen (Marconi B, Filippi GM, Koch G et al. Long-term effects on motor cortical excitability induced by repeated muscle vibration during contraction in healthy subjects. J. Neurol. Sci. 2008; 275: 51-59). S war mit einer einzelnen Stimulationssitzung z.B. eine Nachhaltigkeit von 2 Stunden zu erreichen (Charlton CS, Ridding MC, Thompson PD et al. Prolonged peripheral nerve stimulation induces persistent changes in excitability of human motor cortex. J. Neurol. Sci. 2003, 208: 79-85), was eine Langzeit-Potenzierung der kortikal-plastischen Effekte durch eine längere Behandlungsdauer wahrscheinlich macht (Ragert P, Kalisch T, Bliem B et al. Differential effects of tactile high- and low-frequency stimulation on tactile discrimination in human subjects. BMC Neurosci. 2008; 9:9. 10.1186/1471-2202-9-9). In einer Untersuchung mit gesunden Probanden führte eine 20-minütige rPMS-Anwendung am Unterarm bei 25 Hz zu einer kurzzeitigen fokalen Aktivierung des motorischen Cortex, während eine Frequenz 10 Hz erfolglos blieb (Gallasch E, Christova M, Kunz A et al. Modulation of sensorimotor cortex by repetitive peripheral magnetic stimulation. Front Hum Neurosci. 2015; 9: 407). Interessanterweise verstärkte sich bei 20 Hz die taktile Feinfühligkeit, aber verschlechterte sich bei 1 Hz oder wurden neuromodulatorische Veränderungen bei 15 000 Einzelpulsen (25 Hz rPMS) hervorgerufen, aber nicht bei 6000 Pulsen (10 Hz rPMS) (siehe zitierte Studie Ragert P et al. 2008)

### Studienlage Schlaganfall / spastische Lähmungen

Nach einer rPMS-Applikation im Bereich der sensomotorischen Endaufzweigungen des Unterarms lassen sich die Extensoren bis zu 72 Stunden nach Anwendung mit geringerem Innervationsaufwand weiter und schneller strecken und reduziert sich auch der erhöhte Muskeltonus der Flexoren (Struppler A, Angerer B, Gebhard B: RPMS als Stimulationsmethode zur Rehabilitation sensomotorischer Defizite an Arm und Hand als Folge von zerebralen Läsionen. Neurologie Rehabilitation. 2009; 15: 28-38). Dies dürfte nicht nur auf flüchtige spinale Mechanismen zurückzuführen sein, sondern ist wohl das Ergebnis neuronaler Veränderungen im ZNS (Struppler A, Havel P, Muller-Barna P. Facilitation of skilled finger movements by repetitive peripheral magnetic stimulation (RPMS) - a new approach in central paresis. Neurol Rehabil 2003; 18(1): 69-82). Die Therapieeffekte erstreckten sich dabei auch auf kognitive Funktionen wie auf die spastische Parese, Zielmotorik sowie die kognitive Funktionen wie taktil (Heldmann B, Kerkoff G, Struppler A et al. Repetitive peripheral magnetic stimulation alleviates tactile extinction, in: Neuroreport. 2000;11, S. 3193-3198), räumlich (Liedtke F. Effekte somatosensibler Stimulation auf räumliche Leistungen im personalen Raum. Eine Untersuchung an Gesunden und Patienten mit taktilem Neglect. Psychologische Diplomarbeit an der Katholischen Universität Eichstätt, Lehrstuhl für Klinische- und Neuropsychologie.

Eichstätt. 2004) und visuo-spatial (Struppler A, Havel P: Facilitation of Sensorimotor Performances of Skilled Finger Movements by Repetitive Peripheral Magnetic Stimulation (RPMS) - Cognitive Aspects. In: Dengler R, Kossev AR (Hrsg) Sensorimotor Control - Series I: Life and Behavioural Sciences, IOS Press, Amsterdam- 2001; 57-64), wobei sich damit auch eine Neglect verbessert. Entsprechend verbesserte sich auch das Lageempfinden und reduzierten sich Defizite bei Berührungsreizen > 30 % (Heldmann B, Kerkoff G, Struppler A et al. Repetitive peripheral magnetic stimulation alleviates tactile extinction, in: Neuroreport. 2000;11, S. 3193-3198).

In einer doppelblinden, randomisierten und placebokontrollierten Studie führte eine rPMS-Applikation am gelähmten vorderen Schienbeinmuskel (M. tibialis anterior) zu einer erhöhten Dorsalflexion am Fußgelenk und der isometrischen Kraft. Gleichzeitig nahm der Dehnungswiderstand der plantaren Flexoren ab (Beaulieu LD. Masse-Alarie H, Brouwer B et al. Noninvasive neurostimulation in chronic stroke: a double-blind randomized sham-controlled testing of clinical and corticomotor effects. Top Stroke Rehabil. 2015; 22(1): 8-17). Bei Personen mit einer spastischen Hemiparese sowie einer leicht bis moderat ausgeprägten Spastik nach Schlaganfall oder einer traumatischen Hirnverletzung (26 +/- 71 Wochen bzw. 37 +/- 82 Wochen nach der Verletzung) und einer schweren Lähmung der oberen Extremitäten, die über 2 Wochen 2 x werktäglich mittels rPMS behandelt worden waren, kam es bei der Spastizität zu Kurzzeiteffekten an den Handgelenks- und Langzeiteffekten an den Ellbogen-Flexoren. Die Armmotorik änderte sich in keiner Gruppe signifikant. Allerdings wurde eine sensorische Verbesserung erreicht (Krewer C, Hartl S, Müller F et al. Effects of repetitive peripheral magnetic stimulation on upper limb spasticity and impairment in patients with spastic hemiparesis: a randomized, double-blind, sham-controlled study. Arch Phys Med Rehabil. 2014; 95(6): 1039-1047). Ebenso senkte eine einmalige rPMS, die mit einer manuellen Dehnung kombiniert wurde, signifikant die schwere Spastizität des Handgelenks und der Finger-Flexoren (Werner C, Schrader M, Wernicke S et al. Repetitive peripheral magnetic stimulation (RPMS) in combination with muscle stretch decreased the wrist and finger flexor muscle spasticity in chronic patients after CNS lesion. Int J Phys Med Rehabil. 2016; 4: 352).

In einer weiteren Untersuchung mit Patienten, die nach Schlaganfall an einer Muskelsteifigkeit der Knie-Flexoren bzw. plantaren Flexorengruppe litten, nahm nach 10 rPMS-Behandlungen (10 aufeinanderfolgende Tage) mit gleichzeitiger physiotherapeutischer Unterstützung das Extensionsdefizit nach der ersten Behandlung signifikant ab (Grozoiu L, Savulescu S, Hesse S et al. Repetitive peripheral magnetic stimulation in stroke rehabilitation. - a case study. Int J Soc Sci Humanity. 2016; 6(8): 608-611), wobei die Effekte jeweils 3 Stunden lang anhielten und nach 24 Stunden fast, aber nicht vollständig abgeklungen waren. In einer Folgestudie mit spastischen Patienten der unteren Extremitäten wurde paravertebral (L3 - L4) wieder über 10 aufeinanderfolgende Tage (1 x täglich) behandelt. Sofort nach der ersten Sitzung verminderte sich das Flexionsdefizit auf 20,97 % (Sham-Gruppe) und 13,5 % (Verumgruppe), hielt 2 Stunden an und kehrte nach 24 Stunden wieder zur Baseline zurück. Dabei summierten sich offensichtlich die Effekte nach jeder Sitzung. Die Ashworth-Skala zeigte hier eine signifikante Abnahme in der Verum-, aber nicht in der Shamgruppe (Grozoiu L, Marin AG, Ciobanu I et al. Spastic tone increase reduced using repetitive peripheral magnetic stimulation: pilot study. Int J Soc Sci Humanity. 2017; 7(1): 56-58).

In einer Studie der juvenilen Zentralparese erhielten spastisch gelähmte Kinder 5 rPMS-Anwendungen im tibialen und peronealen Nervenbereich. Dies führte zu einer progressiven Abnahme der Spastik am stärker betroffenen Bein und erreichte nach 3 Anwendungen eine signifikante Ausprägung. Diese anhaltende Reduktion scheint offensichtlich mit den sensorischen Afferenzen und der damit beeinflussten kortikalen Plastizität zusammenzuhängen (Flamand VH, Beaulieu LD. Nadeau L et al. Peripheral magnetic stimulation to decrease spasticity in cerebral palsy. Pediatr Neurol. 2012; 47(5): 345-348).

In einer Untersuchung zur rPMS des kontraläsionalen Handrückens, kam es nach einer 30-minütigen zu einer Reduktion der taktilen Extinktion (Heldmann B, Kerkhoff G, Struppler A et al. Repetitive peripheral magnetic stimulation alleviates tactile extinction. NeuroReport, 2000; 11: 3193-3198), wobei man unter Extinktion das Phänomen versteht, dass Patienten z.B. ihren Arm nicht mehr wahr nehmen und dieser deshalb schlaff herunterhängt. Dieses Ergebnis ließ sich in einer vergleichbaren rPMS-Studie bestätigen (Kerkhoff G, Heldmann B, Struppler A et al. The effects of magnetic stimulation and attentional cueing on tactile extinction. Cortex, 2001; 37, 719-723). Auch verbessert sich durch rPMS der Lagesinn als Zeichen einer verbesserten räumlichen Wahrnehmung (Liedtke F. Effekte somatosensibler Stimulation auf räumliche Leistungen im personalen Raum. Eine Untersuchung an Gesunden und Patienten mit taktilem Neglect. Psychologische Diplomarbeit an der katholischen Universität Eichstätt, Lehrstuhl für Klinische- und Neuropsychologie. Eichstätt. 2004). In einer aktuellen RCT- Studie, die den Einfluss einer rPMS auf die spastische Hemiparese nach Schlaganfall untersuchte, waren zwar nach 20 Behandlungen (2 Wochen/ jeweils 20 Minuten) die Effekte auf die spastischen Effekte entweder gering oder kam es zu keiner Verbesserung der motorischen Funktionen. Dafür verstärkten sich aber die sensorischen Funktionen (Krewer C, Hartl S, Müller F et al. Effects of repetitive peripheral magnetic stimulation on upper-limb spasticity and impairment in patients with spastic hemiparesis: a randomized, double-blind, sham-controlled trial. Arch Phys Med Rehabil. 2014; 95(6): 1039-1047).

### rPMS nach Querschnittslähmung

Bei Querschnittslähmung ist bisher grundsätzlich davon auszugehen, dass eine Heilung im eigentlichen Sinne nicht möglich ist und sich der klinische Befund nach spätestens ein bis zwei Jahren endgültig verfestigt (Rowland JW, Hawryluk GWJ, Kwonn B et al. Current status of acute spinal cord injury pathophysiology and emerging therapies: promise on the horizon. Neurosurgical focus. 2008; 25(5): doi:10.3171/FOC.2008.25.11.E2). Es bleibt trotz allem vorrangiges Ziel, die oftmals schwere spinale Spastik durch geeignete rehabilitative Maßnahmen zu verbessern, Hierbei sind auch noch vorhandene Restfunktionen so zu trainieren, dass sie möglichst erhalten bleiben oder auch durch Aktivierung der systemimmanenten Reorganisationsfähigkeit des lumbalen Rückenmarks zumindest reflektorische Reize an die untere Extremitätenmuskulatur senden (Hubli M, Dietz V. The physiological basis of neurorehabilitation - locomotor training after spinal cord injury. Review. Hubi Dietz J NeuroEngin Rehabil. 2013; 10:5). Durch die Immobilisation und Lähmung kann es auch zur Ausbildung von Kontrakturen kommen, die eventuell sehr schmerzhaft sind. Von der Schmerzsymptomatik sind im Laufe ihres Lebens 70 - 80 % (Wasner G. Zentrale Schmerzen. In: Schmerzmedizin - 1000 Fragen. Bernateck M, Karst M, Sabatowski R, Siebrecht D (Hrsg.). Georg Thieme Verlag, Stuttgart New York. 2. Auflage. 2012) bzw. nach anderen Quellen 26 - 96 % betroffen (Saulino M. Spinal cord injury pain. Phys Med Rehabil Clin N Am. 2014; 25(2): 397-410) Außerdem besteht das grundsätzliche Problem einer starken Muskelschwäche infolge ihrer Atrophie (Thomas CK, Zaidner EY, Calancie B et al. Muscle weakness, paralysis, and atrophy after human cervical spinal cord injury. Exp Neurol. 1997; 148: 414-423).

In einem Review zu den wichtigsten Anliegen Querschnittsgelähmter zählt die Verbesserung ihrer Lebensqualität. Hier stehen vor allem die Blasen- und Darmfunktion, Sexualität, Schmerzen und selbstverständlich auch eine Mobilitätsverbesserung im Vordergrund, wobei 26,7 % der Gelähmten (Paraplegie) die Sexualität am wichtigsten war, gefolgt vom Thema Blasen- und Darmprobleme mit Dysreflexie mit 18 % und einem Zugewinn an Kraft im Oberkörper / Körperstamm / Gleichgewicht mit 16,5 % (Estores IM. The consumer's perspective and the professional literature: What do persons with spinal cord injury want? J. Rehabil. Res. Dev. 2003; 40 (suppl 1): 93-98).

Bei den meisten Patienten sind die spinalen Motoneurone unterhalb der Verletzung noch intakt. Deshalb liegt es nahe, die von ihnen versorgten Muskeln durch eine rPMS an der dorsalen Wirbelsäule zu aktivieren. Denn eine rPMS lässt sich nicht nur im Bereich der nervalen Endaufzweigungen im Muskel (Motorische Endplatte), sondern grundsätzlich auch paraspinal applizieren (Krause P, Straube A. Peripheral repetitive magnetic stimulation induces intracortical inhibition in healthy subjects. Neurol Res. 2008; 30(7): 690-694). Bei lumbosakraler Stimulation kommt es zu einem orthodromen, also eines in Verlaufsrichtung des motorischen Nervs weitergeleiteten Impuls, der sich z.B. mittels Elektroneurogramm als sog. M-Welle abbildet und die Muskeln des Ober- und Unterschenkel sowie des Fußes betrifft (z.B. quadriceps femoris, tibialis anterior, gastrocnemius, extensor digitorus brevis, flexor hallucis brevis, abductor hallucis). Dies entspricht einer Erregung der A-alpha (I) myelinumantelten dicken Nervenfasern bzw. einem Muskelaktionspotential. Neben den damit verbundenen sensomotorischen Verbesserungen, kommt es - wie bei der direkten Applikation an der distalen Muskulatur - zu einer Down-Regulation der hyperaktiven spinalen Erregbarkeit bei Rückenmarkserkrankungen (Krause P, Edrich T, Straube A. Lumbar repetitive stimulation reduces spastic tone increase of the lower limbs. Spinal Cord. 2004; 42(2): 67-72 / Nielsen JF, Sinkjaer T. Long-lasting depression of soleus motoneurons excitability following repetitive magnetic stimuli of the spinal cord in multiple sclerosis patients. Mult Scler. 1997;3(1): 18-30). Damit lässt sich z.B. einer kompletten Gelenkversteifung entgegentreten und somit dem Lebensgefühl neue Anreize geben.

Eine der wichtigste Erkenntnisse zur Relevanz einer rPMS bei Querschnittslähmung ergibt aus dem Forschungsgebiet der epiduralen elektrischen Stimulation (EES) . Hier werden den Patienten im Bereich S2 - L1 Elektroden implantiert, welche per Fernbedienung und elektrischer Impulse die Hinterstränge des Rückenmarks stimulieren. Damit sind in den letzten Jahren einige vielbeachtete Erfolge gelungen, indem Paraplegiker nach jahrelanger Trainingszeit unter externer Impulsgebung und assistierender manueller oder Roboterhilfe wieder einige Schritte gehen konnten (Angeli CA, Degerton VR, Gerasimenko YP et al. Altering spinal cord excitability enables voluntary movements after chronic complete paralysis in humans. Brain. 2014; 137: 1394-1409 / Rejc E, Angeli CA, Bryant N et al. Effects of stand and step training with epidural stimulation on motor function for standing in chronic complete paraplegics. J Neurotrauma. 2017; 34: 1787-1802 / Gill ML, Grahn PJ, Calvert JS et al. Neuromodulation of lumbosacral spinal networks enables independent stepping after complete paraplegia. Nature Med. online Sept 24, 2018, Epub ahead).

Die Bedeutung für den Einsatz einer rPMS ergibt sich hier weniger aus den Steh- und Gehversuchen der Patienten. Vielmehr wird klar, dass das lumbale Rückenmark offensichtlich die Fähigkeit besitzt, ähnlich wie das Repräsentationszentrum in der Hirnrinde (Cortex), eigenständig neue Verschaltungen aufzubauen (Capogrosso M, Wenger N, Raspopovic S et al. A computational model for epidural electrical stimulation of spinal sensorimotor circuits. J Neurosci. 2013; 33(4): 19326-19340). Diese "Plastizität des Rückenmarks" (Courtine G, Song B, Roy R et al. Recovery of supraspinal control of stepping via indirect propriospinal relay connections after spinal cord injury. Nat Med. 2008; 14(1): 69-74), die auch als CPG (Central Pattern Generator) bezeichnet werden, beschreibt ein spezielles Netzwerk von Nervenzellen, die in den unteren Extremitäten selbständige rhythmische Muskelkontraktionen veranlassen können, die beim Stehen oder Gehen eine wichtige Rolle spielen. Das Rückenmark ist also nicht davon abhängig, von übergeschalteten Hirnzentrum Signale zu erhalten, sondern ist selbständig in der Lage, in regelmäßiger Abfolge Aktionspotentiale zu produzieren (O'Shea M. The Brain - A very Short Introduction. Oxford University Press. Oxford 2005). Dabei reichen ungerichtete (ungemusterte) extern applizierte Signale (z.B. EES) aus, um in den unteren Extremitäten Bewegungsmuster im Sinne von alternierenden Steh- und Schwingphasen zu induzieren (Danner SM, Rattay F, Hofstoetter U et al. Locomotor rhythm and pattern generating networks of the lumbar spinal cord: an electrophysiological and computer modeling study. BMC Neurosci. 2013; 14(Suppl 1): P274).

Da wegen der querschnittsbedingt blockierten Reizweiterleitung keine sensorische Perzeption der Bewegungen möglich ist, eine befriedigende Neuorganisation der CPG ("Gehen auf Oberflächen") aber einen visuellen, vestibulären und vor allem auch propriozeptiven Input erfordert (Rossignol S, Dubuc R, Gossard JP: Dynamic sensorimotor interactions in locomotion. Physiol Rev 2006, 86(1):89-154), sind der Plastizität des Rückenmarks allerdings Grenzen gesetzt.

Im Gegensatz zur epiduralen elektrischen Stimulation (EES), die nur mittels Implantation von Elektroden am lumbalen Spinalkanal (Dura mater) wirken kann, durchdringen rPMS-Signale jedwedes organische Gewebe ungehindert. Demzufolge lassen sich durch eine paravertebrale rPMS nicht nur direkte Muskelkontraktionen in den Bein- und Fußmuskeln initiieren, sondern fungieren auch als ein effektiver, nicht-invasiver Stimulus, die massiv gestörten Verschaltungen des Rückenmarks wieder zu einer Reorganisation von Bewegungsmustern anzuregen (Gerasimenko Y, Gorodnichev R, Machueva E et al. Novel and direct access to the human locomotor spinal circuitry. J Neurosci 2010, 30(10):3700-3708). Als "Nebeneffekt" entwickelt dies auch positive Effekte auf den spastischen Muskeltonus (Nielsen JF, Sinkjaer T, Jakobsen J: Treatment of spasticity with repetitive magnetic stimulation; a double-blind placebo-controlled study. Mult Seien 1996, 2(5):227-232 / Nielsen JF, Sinkjaer T: Long-lasting depression of soleus motoneurons excitability following repetitive magnetic stimuli of the spinal cord in multiple sclerosis patients. Mult Scler 1997, 3(1):18-30)

### Studienlage Querschnittlähmung / Spastik u. Muskelaufbau

Aus Tierstudien ist bekannt, dass eine Immobilisation der unteren Extremitäten zu einer 70 - 80 % igen Muskelatrophie führt, wobei die meisten strukturellen Veränderungen schon nach 7 Tagen der Immobilisation beginnen (Qin L, Appell HJ, Chan KM et al. Electrical stimulation prevents immobilization atrophy in skeletal muscle of rabbits. Arch Phys Med Rehabil. 1997; 78: 512-517). Im Falle einer Querschnittsverletzung ist z.B. innerhalb 3 Wochen eine Abnahme des Oberschenkeldurchmessers um 50 % zu messen (Taylor PN, Ewins DJ, Fox B et al. Limb blood flow, cardiac output and quadriceps muscle bulk following spinal cord injury and the effect of training for the Odstock functional electrical stimulation standing system. Paraplegia. 1993; 31: 303-310). In einer Tierstudie führte eine 10-tägige lumbale rPMS im Bereich L3-L5 (20 Hz / 60 Minuten täglich) zu einer Zunahme der Muskelfaserdurchmesser um 20 % (14 - 27 %) (Shimada Y, Sakuraba T, Matsunaga T et al. Effects of therapeutic magnetic stimulation on acute muscle atrophy in rats after hindlimb suspension. Biomed Res. 2006; 27(1): 23-27).

In einer Untersuchung zum spastische Syndrom nach Querschnittslähmung und einer rPMS der lumbalen Nervenwurzeln (L3-L4) reduzierte sich der spastische Tonus über einen Zeitraum von 4 - 24 Stunden nach Einzelstimulation. Dabei war der Effekt an der kontralateralen Extremität etwas stärker ausgeprägt (Krause P, Edrich T, Straube A. Lumbar repetitive magnetic stimulation reduces spastic tone increase of the lower limbs. Spinal Cord. 2004; 42(2): 67-72). Frühere rPMS-Studien haben hier bereits gezeigt, dass rPMS die tonische Komponente der Beugespastik deutlich vermindert, ganz egal, ob es sich um eine zentrale Lähmung (Schlaganfall) oder eine Querschnittsverletzung handelt (Struppler A, Havel P, Muller-Barna P et al. A new method for rehabilitation of central palsy of arm and hand by peripheral magnetic stimulation. Neurol Rehabil 1997; 3: 145-158 / Struppler A, Jacob C, Müller-Barna P et al. Eine neue Methode zur Frührehabilitation zentralbedingter Lähmungen von Arm und Hand mittels peripherer Magnetstimulation. Zeitschrift Elektroenze-phalo Elektromyo Verwand Gebiete. 1996; 27: 151-157).

### Studienlage Querschnittslähmung / neurogene Blase

Nachdem die Behebung von Blasenstörungen im Ranking der wichtigsten lebensqualitätsverbessernden Ziele von Querschnittsgelähmten an vorderster Stelle steht (Simpson LA, Eng JJ, Hsieh JT et al. The health and life priorities of individuals with spinal cord injury: a systematic review. J Neurotrauma. 2012; 29, 1548-1555), wurde in einer "Proof-of-Concept-Studie" die Relevanz einer rPMS-Behandlung untersucht (Niu T, Bennett, CJ, Keller TL et al. A proof-of-concept study of transcutaneous magnetic spinal cord stimulation for neurogenic bladder. Sci Reports. 2018; 8: 12549). Denn Querschnittspatienten können nicht willentlich urinieren und entleeren ihre Blase meist durch Selbst-Katherisation - mit den bekannten Folgen einer ernst zu nehmenden Harnblaseninfektion (Nicolle LE. Urinary tract infections in patients with spinal injuries. Curr Infect Dis Rep. 2014; 16(1): 390). Dies erfolgte unter der Hypothese auf Basis einer Studie zur Tetraplegie, nach der das (unverletzte) spinale Netzwerk das Potential besitzt, eine Vielzahl koordinierender Bewegungen selbständig, also ohne Input des ZNS auszuführen (Lu DC, Edgerton VR, Modaber M et al. Engaging cervical spinal cord networks to reenable volitional control of hand function in tetraplegic patients. Neurorehabil Neural Repair. 216; 30(10): 951-962). Um die optimale Frequenzeinstellung zu finden, erhielten fünf Querschnitts-Patienten mit einer Detrusorsphinkter-Dyssynergie über 2 Wochen (2 x täglich) insgesamt 32 rPMS-Applikationen mit 1 Hz und 30 Hz jeweils für eine Woche paraspinal. Dabei ließ sich mit einer Frequenz von 1 Hz der Harnröhren-Druck reduzieren und gleichzeitig den Detrusordruck verstärken. In der darauf folgenden Behandlungsphase (16 Wochen) erfolgte eine Frequenzapplikation von 1 Hz. In einer Follow-up-Phase wurde bei den Patienten dann noch eine "Sham- bzw. Placebophase" von 6 Wochen angehängt (Intensität < 5 %) oder solange, bis sich die zuvor erreichten positiven Effekte wieder zurückgebildet hatten. Ergebnis: Nach 16 Wochen wurde die Blasenfunktion bei allen Patienten zum Teil wieder hergestellt. Vier der fünf Probanden (80 %) konnten die Häufigkeit der Selbst-Katherisierung um mindestens 50 % senken. Ein Patient (20 %) war in der Lage, wieder selbstständig (ohne Katheter) zu urinieren und ein weiterer benötigte nur eine Katherisation pro Tag.

In einer weiteren Untersuchung verbesserte sich die Inkontinenzsymptomatik wegen Detrusorsphinkter-Dyssynergie nach lumbaler rPMS ab einer Blasenfüllung > 200 ml (Brodack PP, Bidair M, Joseph A et al. Magnetic stimulation of the sacral roots. Neurourol Urodyn. 1993; 12(6): 533-540).

### Querschnittslähmung / sexuelle Dysfunktion

Obwohl 80 % aller Querschnittsgelähmten von einer erektilen Dysfunktion betroffen sind, bleibt der autonome Spinalreflex nach lumbalen Verletzungen grundsätzlich erhalten. Reflektorische Erektionen durch Manipulation am Penis sind damit bei etwa drei Viertel aller Patienten (Kim MS, Park BG, Jung CJ. A study of erection in spinal cord injured patients. J Korean Acad Rehabil Med. 1996; 20(3): 9 / Horne MW, Paull DP, Munro D. Fertility studies in the human male with traumatic injuries of the spinal cord and cauda equine. New Engl J Med. 1948; 237: 959-961) und damit auch durch rPMS auslösbar, allerdings mit der Einschränkung, dass der Patient (bei der kompletten Querschnittslähmung) meist nichts davon verspürt. Zudem hat die Erektion keinen langen Bestand, da weitere zentralnervöse Reize fehlen. Auch ist die Ejakulation meist behindert, wobei sehr wohl Studienbeweise zur Zeugungsfähigkeit existieren (Raymond CA. New use for old method of inducing ejaculation may give hope of fatherhood to some spinal cord injured men. JAMA 1987; 258(6): 743-744 / Bennett CJ, Ayers JWT, Randolph JF et al. Electroejaculation of paraplegic males followed by pregnancies. Fert Steril. 1987; 48: 1070-1072). Einer hier notwendigen künstlichen Befruchtung ist allerdings immer erst das mühsame Verfahren einer Elektroejakulation vorzuschalten (Randolph JF Jr, Ohl DA, Bennett CJ et al. Combined electroejaculation and in vitro ferilization in the evaluation and treatment of anejaculatory infertility. J Vitro Fert Embryo Transf. 1990; 7(1): 58-62), die durch Stimulation des Plexus pelvicus und der Prostata mittels Analelektroden erfolgt. Aber auch dann keine die Insemination durch eine zu geringe Qualität des Spermas scheitern (Linsenmeyer TA, Perkash I. Review article: Infertility in men with spinal cord injury. Arch Phys Med Rehabil. 1991;72: 747-754). wobei diese sich durch wiederkehrende Stimulationsreize wieder erholen kann (Francois N, Maury M, Jouannet D et al. Electroejaculation of a complete paraplegic followed by pregnancy. Paraplegia 1978; 16:248-51 / Brindley GS. Physiology of erection and management of paraplegic infertility. In Haregrave TB, ed. Male Infertility. Berlin: Verlag Springer 1983;261-79).

Hieraus ist ein Angriffspunkt für eine rPMS zu formulieren, da eine Magnetstimulation über ihre Tiefenwirkung und Intensität einer analen Elektrostimulation ebenbürtig (Rossini PM, arker AT, Berardelli A et al. Noninvasive electrical and magnetic stimulation of the brain, spinal cord and roots: basic principles and procedures for routine clinical applacation. Report of an IFCN committee. Electroenceph Clin Neurophydiol. 1994; 91: 79-92), wenn nicht gar überlegen ist. Abgeleitet von einer analen Elektrostimulation der Prostata (Levin RJ. Prostate-induced orgasms: A concise review illustrated with a highly relevant case study. Clin Anatomy. 2018; 31: 81-85) ist auf jeden Fall davon auszugehen, dass eine Prostatareaktion erfolgt. Allerdings mit der kleinen Einschränkung bei Läsionen im Sakralbereich, weil hier eine retrograde Ejakulation wahrscheinlich ist (Sonksen J, Biering-Sorensen F, Kristensen JK. Ejaculation induced by penile vibratory stimulation in men with spinal cord injuries. The importance of the vibratory amplitude. Paraplegia. 1994; 32: 651-660).

### Querschnittslähmung / Experimentelle Erkenntnisse

Untersuchungen an gesunden Probanden haben ergeben, dass sich der Beckenboden bei sämtlichen Bewegungen des Körpers immer co-kontrahiert und oftmals die prozentual aktivste Muskelgruppe ist im Vergleich zu den vordergründig eigentlich direkt betroffenen Muskeln (Hodges PW, Eriksson AE, Shirley D et al. Intra-abdominal pressure increases stiffness of the lumbar spine. J Biomech. 2005; 38:1873-80 / Schulte-Frei B. Sport und Bewegungstherapie für den weiblichen Beckenboden. Alltagsrelevanz, Analyse und Therapie unter besonderer Berücksichtigung der neuromuskulären Ansteuerung. Disssertation. Köln. 2006), und das sogar noch in einem antizipativen "Feedforward-Reflex", also Millisekunden, bevor die eigentliche Bewegung erfolgt (Allison GR, Morris SL, Lay B. Feedforward responses of transversus abdominis are directionally specific and act asymmetrically: implications for core stability theories. J Orthop Sports Phys Ther. 2008; 38(5): 228-237). Gleichzeitig scheinen sich aber auch die Bauchmuskeln (Mm. transversus abdominis / obliquus) in einer Vorspannung vor der geplanten Bewegung zusammenzuziehen (Hodges PW, Richardson CA. Contraction of the abdominal muscles associated with movement of the lower limb. Phys Ther 1997; 77: 132-144). Nachdem aber mit jeder Anspannung der Bauchmuskulatur auch eine Co-Kontraktion des Beckenbodens entsteht (Sapsford R, Hodges PW, Richardson CA et al. Co-activation of the abdominal and pelvic floor muscles during voluntary exercices. Neuourol Urodynam 2001; 20: 31-42), bleibt es unklar, in welcher zeitlichen Reihenfolge dies geschieht.

In einem Feldversuch mit dem rPMS-System Pelvicenter, mit dem eine Stimulation der Beckenbodenmuskulatur sowie der sonstigen Beckengürtel- und tiefen unteren Rückenmuskeln möglich ist, kontrahierte sich bei einer Frequenz von 5 Hz die Bauchmuskulatur, während sich das eigentliche Zielgebiet des Beckenbodens erst ab einer Frequenz von 10 - 25 Hz bemerkbar machte. Physiologisch reagiert ein Muskel bei einer Frequenz von 5 Hz immer nur mit Einzelzuckungen, die ab einer Frequenz von etwa 15 - 20 Hz immer mehr in eine Dauerkontraktion, d.h. einen für die Muskelstimulation günstigen Tetanus übergehen. Unter der Maßgabe einer hier vorliegenden inkompletten Querschnittslähmung, bei der die Lage-, Vibrations- und Berührungsempfindungen grundsätzlich noch vorhanden sind und es klar ist, dass die Reichweite der applizierten rPMS nicht bis in die Bauchmuskulatur reicht, können abdominelle Muskelkontraktionen als spinaler Reflex zu deuten sein, der seinen Ursprung aus dem Beckenboden nimmt. Dort reicht aber die Frequenzhöhe von 5 Hz nicht aus, eine komplette Kontraktion des Beckenbodens in Gang zu setzen. Denn nach der Integraltheorie nach Petros (Petros PE, Ulmsten Ul. An integral theory and its method for the diagnosis and management of female urinary incontinence. Scand J Urol Nephrol Supp. 1993; 153:1 - 93), ist der Beckenboden als komplette funktionelle Einheit zu sehen, die sich aus kontinenzerhaltenden Gründen im Verbund zusammenzieht. Der Gesamtverbund reagiert aber erst ab einem Muskeltetanus, während Einzelzuckungen nur inferiore Teile des Beckenbodens aktivieren können. Darauf könnte auch eine Studie mit gesunden Probanden hindeuten, bei der eine 20-minütige rPMS-Anwendung am Unterarm bei 25 Hz zu einer kurzzeitigen fokalen Aktivierung des motorischen Cortex führte, aber eine Frequenz 10 Hz erfolglos blieb Gallasch E, Christova M, Kunz A et al. Modulation of sensorimotor cortex by repetitive peripheral magnetic stimulation. Front Hum Neurosci. 2015; 9: 407). Obwohl es diesem hypothetische Erklärungsmodell noch an Konkludenz und Beweiskraft mangelt, bleibt festzuhalten, dass dieses Phänomen mit einem Feed-Forward-Reflex im Sinne einer muskulären Vorspannung zusammenhängt.

### rPMS / Multiple Sklerose

Durch den völligen Funktionsverlust motorischer Nerven entwickelt sich bei fortgeschrittener MS das Vollbild einer Querschnittslähmung.

### Studienlage

Bei mehr als 2/3 aller fortgeschrittenen MS-Fälle treten moderate bis schwere Spastiken auf (Zettl UK, Henze T, Essner U et al. Bürden of disease in multiple sclerosis patients with spasticity in Germany: mobility improvement study (Move I). Eur J Health Econ. 2013; 15(9):953-966).

In einer der ersten Untersuchungen zur rPMS-Behandlung von Spastiken bei MS-Patienten, kam es zu einer Abnahme des Muskeltonus mit einer Haltedauer von 1 Woche (Nielsen JF, Sinkjaer T, Jakobsen J. Treatment of spasticity with repetitive magnetic stimulation; a double-blind placebo-contolled study. Mult Scler. 1996; 2: 227-232). Dies wurde durch eine neuere Studie (1 Hz / lumbale Applikation) bestätigt, allerdings mit dem Unterschied, dass die Anti-Spastikeffekte noch mindestens einen Monat nach Therapieende wirkten (Serag H, Abdelgawad D, Emara T et al. Effects of para-spinal repetitive magnetic stimulation on Multiple Sclerosis related spasticity. Int J Phys Med Rehabil. 2014; 2(6): 1000242).

In einer weiteren Studie mit Spastiken der unteren Extremitäten erfolgte die rPMS-Behandlung im Bereich Th8 (12 Hz / 8 sec - 22 sec Pause / 30 Minuten / 40 - 65 % der maximale Stimulationsintensität). Sowohl in der Selbstbeurteilung der Patienten hinsichtlich täglicher Aktivitäten, als auch nach klinischen Spastik-Scores kam es dabei zu einer signifikanten Verbesserung (globale Reduktion der Ashworth-Scores / einer signifikanten Abnahme der Soleus-Dehnungs-Reflex-Amplitude bzw. Hyperreflexie sowie eine Zunahme des Geschwindigkeits-Schwellenwerts) (Nielsen JF, Klemar B, Hansen HJ. A new treatment of spasticity with repetitive magnetic stimulation in multiple sclerosis. J Neurol Neurosurg Psychiatry. 1995; 58(2): 254-255).

Eine thorakale rPMS bei MS-Patienten mit einer Beinspastik ( 5 Minuten / 25 Hz) führte zu einer lang anhaltenden Abnahme des sog. H-Reflex (70 % gegenüber einer kurzen Erststimulation) am M. soleus, wobei man hier vermutet, dass dies mit einer Erniedrigung der synaptischen Transmission zusammenhängt (Nielsen JF, Sinkjaer T. Long-lasting depression of soleus motoneurons excitability following repetitive magnetic stimuli of the spinal cord in multiple slerosis patients. Scler. 1997; 3(1): 18-30. Beim H-Reflex handelt es sich um das elektrische Korrelat des Muskeleigen-reflexes bzw. ist eine motorische Spätantwort nach Stimulation eines gemischten oder motorischen Nervs. Dieselben Autoren hatten auch schon zuvor MS-Patienten behandelt (doppelblind, placebokontrolliert) mittels rPMS an den Spinalwurzeln (thorakal / 2 x täglich über 7 aufeinanderfolgende Tage). Nach Therapieende zeigte sich (Ashworth-Skala) eine maximale Abnahme des Widerstands der Hüft-, Knie- und Knöchel-Flexoren und - Extensoren beider Beine, was sich zwischen dem 8. und 16. Tag zu Baseline-Werten reduzierte (Nielsen JF, Sinkjaer T, Jacobsen J. Treatment of spasticity with repetitive magnetic stimulation; a double-blind placebo-controlled study. Mutl Scler. 1996; 2(5): 227-232)

Abgeleitet von der protektiven Bedeutung bewegungstherapeutischer Maßnahmen und eines rehabilitativen Krafttrainings, das MS-Betroffenen nicht nur einen erheblichen Kraftgewinn bringt, sondern auch eine Fatigue verbessert und damit auch Stimmung und Lebensqualität, liegt es nahe, rehabilitativ eine rPMS einzusetzen. Denn die rPMS besitzt ein erhebliches Potential in der Neuromodulation und dem Training schwacher oder geschädigter Muskeln (Swallow EB, Gosker HR, Ward KA et al. A novel technique for nonvolitional assessment of quadriceps muscle endurance in humans. J Appl Physiol. 2007; 103(3): 739-746), zumal sie wegen ihrer Tiefenwirkung auch Muskeln erfasst die z.B. für eine Elektrostimulation unerreichbar sind (Millet GY, Bachasson D, Temesi J et al. Potential interests and limits of magnetic and electrical stimulation techniques to assess neuromuscular fatigue. Neuromuscul. Disord. 2012, 22 (Suppl. S3), S181-S186), oder wegen krankheitsbedingter Einschränkungen nicht trainiert werden können. Bei einer Fatigue handelt es sich mit 50 - 83 % % (Kirsten M, Fisk JD, Patten SB et al. Fatigue and comorbidities in Multiple Sclerosis. Int J MS Care.2016; 18(2): 96-104) um das häufigste Symptom von MS-Erkrankten und wird von ihnen als Erschöpfung, Ermüdung und Energielosigkeit wahrgenommen.

### rPMS / Angiogenese / Blutzirkulation / Oxygenierung

Zwar gibt es noch keine größeren Erkenntnisse zum Einfluss einer rPMS auf Organe oder sonstiges Weichteilgewebe. Doch ist zumindest von einer regionalen Steigerung der Blutzirkulation und Oxygenierung auszugehen (Mesquita RC, Faseyitan OK, Turkeltaub PE et al. Blood flow and oxygenation changes due to low-frequency repetitiv transcranial magnetic stimulation of the cerebral cortex. J Biomed Optics. 2013; 18(6): 067006). Auch ist nach paravertebraler rPMS ein Anstieg von eNOS (endotheliale Stickstoffmonoxid-Synthetase) sowie des Proteins AKT in den unteren Extremitäten (M. quadriceps femoris) nachzuweisen (Lee D. Beom J, OH BM et al. Effect of magnetic stimulation in spinal cord on limb angiogenesis and implication: a pilot study. Ann Rehabil Med. 2012; 36: 311-319). eNOS katalysiert die Bildung von Stickstoffmonoxid, was durch die dadurch bedingte Erschlaffung der glatten Gefäßmuskelzellen und einer Thrombozytenaggregationshemmung eine Durchblutungssteigerung bedingt. AKT wiederum ist wiederum ein Mediator der Angiogenese, was durch eine Aktivierung von VEGF (Vascular Endothelial Growth Factor) erfolgt (Jiang BH, Liu Z. AKT signaling in regulating angiogenesis. Curr Cancer Drug Targets. 2008; 8(1): 19-26). Dabei sind die Wirkungen frequenzabhängig, d.h. entsprechen bei 20 Hz einem Mehrfachen einer 1-Hz-Stimulation. Aus den angiogenetischen Effekten ist wiederum ein Potential zur Lymphangiogenese abzuleiten.

### rPMS / Schmerztherapie

Nachdem eine nur 5-minütige paraspinale Magnetstimulation zu einem lang anhaltenden (30 - 40 Minuten) und starken (80 - 90 % der maximal möglichen Wirkung) antinozizeptiven Effekt sowohl bei mechanisch, als auch hitzeverursachten Schmerzen führte (Lin VW, Hsiao I, Kingery WS. High intensity magnetic stimulation over the lumbosacral spine evokes antinociception in rats. Clin Neurophysiol. 2002; 113(7): 1006-1012) , könnte sich nach entsprechenden Humanversuchen ein weiteres zukunftsträchtiges Einsatzgebiet für die rPMS ergeben. Besonders interessant ist hierbei, dass dem zugrundeliegenden Effekt offensichtlich ein (körpereigener) opioider Mechanismus zugrunde liegt.

Gemäß der Erfindung können an den Seitenteilen bzw. Armauflagen Haltegriffe zum Ziehen durch den Patienten angeordnet sein, so dass man die Muskelverstärkung messbar machen kann.

Es konnte festgestellt werden, dass bei einer Stimulationsfrequenz von etwa 5 Hz die äußeren Muskeln, insbesondere auch die Bauchmuskeln, stark kontrahiert werden. Der innen liegende Pelvis-Muskel zeigt erst bei einer Stimulationsfrequenz von ca. 10 bis 25 Hz. eine Kontraktion.

Weiter werden über ein Display die Stellung der Magnetspulen und die Leistungssteigerung über die beiden Handgriffe angezeigt.

Um das anzeigen zu können, muss die Körpergröße des Benutzers gemessen und die Position der Magnetspulen in Bezug auf den Körper genau angezeigt werden. Dafür können im Kopfteil und im Fußbereich Messsensoren angebracht sein, so dass die Körpergröße ermittelt werden kann. Die Steigerung der Muskelkraft kann auf dem Display beispielsweise mit einem Balkendiagramm angezeigt werden. Hierbei kann ein Vorher-Nachher Vergleich angezeigt werden. Dadurch wird der Benutzer motiviert, die Anwendungsstärke und Anwendungsdauer zu steigern.

Der Behandlungsstuhl ist insbesondere zur Durchführung einer repetitiven transpelvinen Magnetstimulation TPM oder einer repetitiven peripheren Magnetstimulation rPMS oder einer repetitiven transkraniellen Magnetstimulation rTMS geeignet.

Der Erfindungsgegenstand der vorliegenden Erfindung ergibt sich nicht nur aus dem Gegenstand der einzelnen Patentansprüche, sondern auch aus der Kombination der einzelnen Patentansprüche untereinander.

Alle in den Unterlagen, einschließlich der Zusammenfassung offenbarten Angaben und Merkmale, insbesondere die in den Zeichnungen dargestellte räumliche Ausbildung, werden als erfindungswesentlich beansprucht, soweit sie einzeln oder in Kombination gegenüber dem Stand der Technik neu sind.

Im Folgenden wird die Erfindung anhand von lediglich einen Ausführungsweg darstellenden Zeichnungen näher erläutert. Hierbei gehen aus den Zeichnungen und ihrer Beschreibung weitere erfindungswesentliche Merkmale und Vorteile der Erfindung hervor.
- Figur 1: zeigt eine perspektivische Darstellung eines TPM / rPMS-Behandlungsstuhls.
- Figur 2: zeigt eine perspektivische Darstellung eines TPM / rPMS-Behandlungsstuhls mit verschobenem Seitenteil.
- Figur 3: zeigt eine perspektivische Darstellung einer anderen Ausgestaltung eines TPM / rPMS-Behandlungsstuhls.

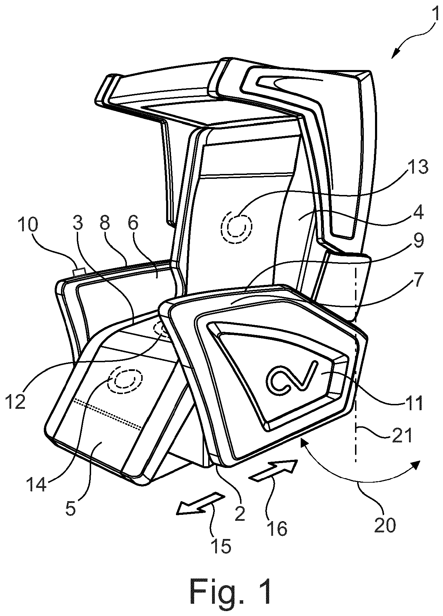

Gemäß Figur 1 besteht der TPM / rPMS-Behandlungsstuhl 1 aus einem Grundgestell 2, auf welchem eine Sitzfläche 3 angeordnet ist. Am hinteren Ende der Sitzfläche ist eine Rückenlehne 4 vorgesehen, die vorzugsweise schwenkbar an der Sitzfläche 3 angeordnet ist. Das Grundgestell steht fest auf dem Boden des Behandlungsraumes.

Am vorderen Ende der Sitzfläche 3 ist vorzugsweise eine Beinstütze 5 angeordnet, die in Bezug zur Sitzfläche 4 verschwenkbar sein kann, so dass sie horizontal zur Sitzfläche 3 oder in einem Winkel zur Sitzfläche 3 eingestellt werden kann. Die Beinstütze 5 kann jedoch auch fest mit der Sitzfläche 3 verbunden sein. Die Sitzfläche 3 wird seitlich von zwei zueinander parallelen Seitenteilen 6, 7 eingefasst, wobei jedes Seitenteil 6, 7 eine Armauflage 8, 9 aufweist. In einer oder in beiden Armauflagen 8, 9 kann ein Bedienungspanel 10, 11 angeordnet ist.

Es kann beispielsweise vorgesehen sein, dass über das Bedienungspanel 10 der Behandler bestimmte Parameter eingibt oder einliest, während am Bedienungspanel 11 der Benutzer individuelle Einstellungen während der Behandlung vornimmt.

In der Sitzfläche 3 ist mindestens eine Magnetspule 12 angeordnet. Die Magnetspule 12 ist in die Sitzfläche 3 eingearbeitet und von außen nicht sichtbar.

Die Magnetspule 12 ist Längsrichtung der Sitzfläche 3 verschiebbar und feststellbar angeordnet, so dass die Position der Magnetspule 12 in der Sitzfläche 3 auf den jeweiligen Benutzer und die Behandlung einstellbar ist.

Auch in der Rückenlehne 4 ist mindestens eine weitere Magnetspule 13 angeordnet. Die Magnetspule 13 ist in die Rückenlehne 4 eingearbeitet und von außen nicht sichtbar. Die Magnetspule 13 ist in Längsrichtung der Rückenlehne 4 verschiebbar und feststellbar angeordnet, so dass die Position der Magnetspule 13 in der Rückenlehne 4 auf den jeweiligen Benutzer und die Behandlung einstellbar ist.

Um die Verschiebungslage dieser Magnetspulen 12, 13 in der Sitzfläche 3 und der Rückenlehne 4 von außen her optisch erkennbar zu gestalten, kann es vorgesehen sein, dass an der Armauflage 8, 9 eine Positionsanzeige vorgesehen ist, welche die jeweilige Position der Magnetspulen 12, 13 anzeigt.

Ferner kann es vorgesehen sein, dass ebenfalls in der Beinstütze 5 eine Magnetspule 14 angeordnet ist. Auch diese Magnetspule 14 kann vorzugsweise in Längsrichtung der Beinstütze 5 verschiebbar und feststellbar angeordnet sein, so dass die Position der Magnetspule 14 in der Beinstütze 5 auf den jeweiligen Benutzer und die Behandlung einstellbar ist. Eine optische Anzeige der Position der Magnetspule 14 kann vorgesehen sein.

Aufgrund der relativ hohen Seitenteile 6, 7 und der Beinstütze 5 ist ein Einsteigen des Benutzers auf die Sitzfläche 3 schwierig. Insbesondere körperlich beeinträchtigte und körperbehinderte Personen brauchen zum Platznehmen auf der Sitzfläche 3 oftmals fremde Hilfe. Auch ist es z.B. für Rollstuhlfahrer schwierig, vom Rollstuhl auf die Sitzfläche 3 des Behandlungsstuhls 1 und wieder zurück zu wechseln.

Um den Einstieg auf den Behandlungsstuhl zu erleichtern ist es erfindungsgemäß vorgesehen, dass zumindest eine Seitenwand 7 in den Pfeilrichtungen 15, 16 verschiebbar ist oder in Pfeilrichtung 20 verschwenkbar ist.

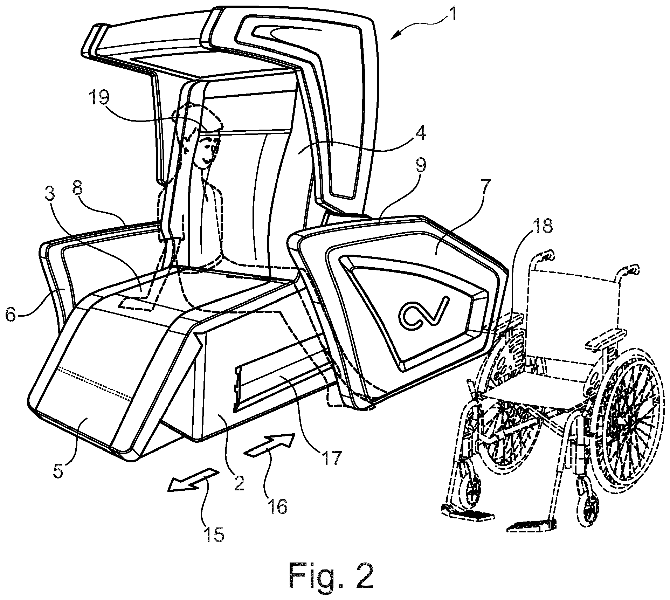

Figur 2 zeigt den Behandlungsstuhl 1 nachdem das Seitenteil 7 in Pfeilrichtung 16 nach hinten verschoben wurde. Das Verschieben des Seitenteils 7 kann manuell und/oder automatisch mittels eines Antriebs erfolgen, der z. B. am vom Benutzer am Bedienungspanel 10, 11 des Behandlungsstuhls 1 betätigt wird.

Durch das nach hinten verschobene Seitenteil 7 ist ein seitlicher Einstieg eines Benutzers 19 und ein Platznehmen auf der Sitzfläche 3 des Behandlungsstuhls 1 bequem möglich. Selbst Rollstuhlfahrer können seitlich bis an die Sitzfläche 3 des Behandlungsstuhls 1 hinfahren und relativ einfach von der Sitzfläche des Rollstuhls auf die Sitzfläche 3 des Behandlungsstuhls 1 und wieder zurück wechseln.

Das verschiebbare Seitenteil 7 ist vorzugsweise in einer Führungsschiene 17 verschiebbar gelagert, die am Grundgestell 2 des Behandlungsstuhls 1 befestigt ist. An der Unterseite des Seitenteils 7 können vorzugweise eine oder mehrere Stützrollen 18 angeordnet sein, die beim verschieben des Seitenteils 7 auf dem Boden abrollen. Diese Stützrolle 18 nimmt die Last des Seitenteils 7 teilweise auf und erleichtert so das Verschieben des Seitenteils 1.Ferner kann sich der Benutzer 19 während des Platznehmens auf dem Behandlungsstuhl 1 auf dem Seitenteil 7 mit seinem Körpergewicht abstützen, wobei diese Last durch die Stützrolle 18 und die Führungsschiene 17 ebenfalls aufgenommen wird.

In Figur 1 ist es angedeutet, dass anstelle einer Verschiebung des Seitenteils 7 in den Pfeilrichtungen 15, 16 alternativ ein Verschwenken des Seitenteils 7 in Pfeilrichtung 20 erfolgen kann.

In dieser alternativen Ausgestaltung der Erfindung ist das Seitenteil 7 um eine etwa senkrecht verlaufende Schwenkachse 21 schenkbar am Grundgestell gelagert und kann in Pfeilrichtung nach hinten und wieder zurück in die Ausgangsstellung verschwenkt werden. Das Seitenteil 7 kann somit ähnlich einer Autotür, jedoch mit einer Öffnungsbewegung nach hinten, geöffnet werden, so dass der seitliche Zugang zur Sitzfläche 3 des Behandlungsstuhls 1 freigegeben wird.

Auch in dieser Ausgestaltung kann die Schwenkbewegung des Seitenteils 7 durch eine am Seitenteil angeordnete Stützrolle erleichtert werden, wobei die Stützrolle die Last des Seitenteils 7 teilweise aufnimmt.

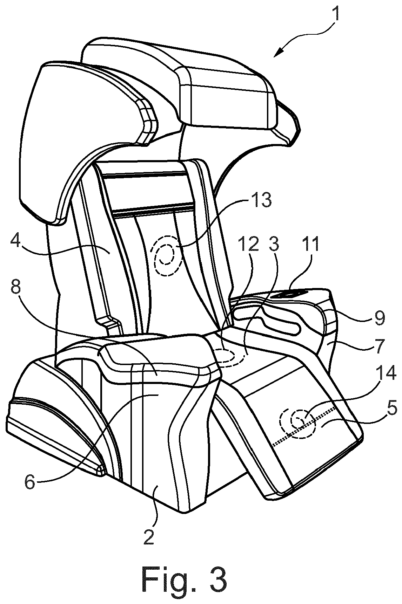

Figur 3 zeigt eine perspektivische Darstellung einer anderen Ausgestaltung eines TPM / rPMS-Behandlungsstuhls. Dieser Behandlungsstuhl 1 weist die im Zusammenhang mit den Figuren 1 und 2 beschriebenen Funktionen auf, hat aber ein geändertes Design.

### Zeichnungslegende

- 1: Behandlungsstuhl
- 2: Grundgestell
- 3: Sitzfläche
- 4: Rückenlehne
- 5: Beinstütze

- 6: Seitenteil
- 7: Seitenteil
- 8: Armauflage
- 9: Armauflage
- 10: Bedienungspanel
- 11: Bedienungspanel
- 12: Magnetspule
- 13: Magnetspule
- 14: Magnetspule
- 15: Pfeilrichtung
- 16: Pfeilrichtung
- 17: Führungsschiene
- 18: Stützrolle
- 19: Benutzer
- 20: Pfeilrichtung
- 21: Schwenkachse

## Patentansprüche

1. Behandlungsstuhl (1) für die transpelvine Magnetstimulation von Körpergewebe, bestehend aus einem Grundgestell (2) mit einer Sitzfläche (3) und einer Rückenlehne (4), sowie zwei Seitenteilen (6, 7) mit Armauflagen (8, 9), wobei in der Sitzfläche (3) und/oder der Rückenlehne (4) mindestens eine Magnetspule (12, 13) angeordnet ist, wobei zumindest eines der beiden Seitenteile (6, 7) verschiebbar oder klappbar am Grundgestell (2) angeordnet ist, derart, dass ein seitlicher Zugang zur Sitzfläche (3) des Behandlungsstuhls (1) freigegeben wird, **dadurch gekennzeichnet, dass** an den Seitenteilen (6, 7) oder Armauflagen (8, 9) Haltegriffe zum Ziehen durch den Benutzer angeordnet sind, die zur Messung einer Steigerung der Muskelkraft aufgrund der Magnetstimulation ausgebildet sind, dass die Magnetspulen (12, 13) in Längsrichtung und/oder Querrichtung der jeweiligen Sitzfläche (3) und/oder Rückenlehne (4) verschiebbar angeordnet sind, und dass ein Bedienpanel (10, 11) mit einem Display zur grafischen Anzeige der Stellung der Magnetspulen (12, 13) und der Leistungssteigerung über die Haltegriffe ausgebildet ist.

2. Behandlungsstuhl nach Anspruch 1, **dadurch gekennzeichnet, dass** das Seitenteil (6, 7) nach hinten in Richtung der Rückenlehne (4) verschiebbar ist und dadurch die Sitzfläche (3) seitlich freigibt.

3. Behandlungsstuhl nach einem der Ansprüche 1 oder 2, **dadurch gekennzeichnet, dass** das Seitenteil (7) am Grundgestell (1) angeordnet und in einer Führungsschiene (17) entlang des Grundgestells (2) verschiebbar gelagert sind.

4. Behandlungsstuhl nach Anspruch 1, **dadurch gekennzeichnet, dass** das Seitenteil (6, 7) abklappbar am Grundgestell (1) angeordnet ist.

5. Behandlungsstuhl nach Anspruch 1 oder 4, **dadurch gekennzeichnet, dass** das Seitenteil (6, 7) um eine am hinteren Teil des Grundgestells (1) angeordnete vertikale Schwenkachse (21) nach hinten verschwenkbar ist und dadurch die Sitzfläche (3) seitlich freigibt.

6. Behandlungsstuhl nach einem der Ansprüche 1 bis 5, **dadurch gekennzeichnet, dass** das Seitenteil (6, 7) an seiner dem Boden zugewandten Unterseite mindestens eine Stützrolle (18) aufweist, die beim Verschieben oder dem Schwenken des Seitenteils (6, 7) auf dem Boden abrollt.

7. Behandlungsstuhl nach einem der Ansprüche 1 bis 6, **dadurch gekennzeichnet, dass** er eine an die Sitzfläche (3) anschließende und relativ zur Sitzfläche (3) verschwenkbare Beinstütze (5) aufweist.

8. Behandlungsstuhl nach Anspruch 7, **dadurch gekennzeichnet, dass** im Bereich der Beinstütze (5) eine Magnetspule (14) angeordnet ist.

9. Behandlungsstuhl nach einem der Ansprüche 7 oder 8, **dadurch gekennzeichnet, dass** die Magnetspule (14) in Längsrichtung und/oder Querrichtung der Beinstütze (5) verschiebbar angeordnet ist.

10. Behandlungsstuhl nach einem der Ansprüche 8 oder 9, **dadurch gekennzeichnet, dass** das Bedienpanel mit dem Display ausgebildet ist, um die Stellung der Magnetspule (14) grafisch anzuzeigen.

11. Behandlungsstuhl nach einem der Ansprüche 1 bis 10, **dadurch gekennzeichnet, dass** er Messsensoren zur Ermittlung der Körpergröße des Benutzers aufweist.

12. Behandlungsstuhl nach einem der Ansprüche 1 bis 11, **dadurch gekennzeichnet, dass** er zur Durchführung einer repetitiven transpelvinen Magnetstimulation TPM oder einer repetitiven peripheren Magnetstimulation rPMS oder einer repetitiven transkraniellen Magnetstimulation rTMS ausgebildet ist.

## Claims

1. Treatment chair (1) for transpelvic magnetic stimulation of body tissue, consisting of a base frame (2) with a seat area (3) and a backrest (4), and two side parts (6, 7) with arm supports (8, 9), wherein at least one magnetic coil (12, 13) is arranged in the seat area (3) and/or the backrest (4), wherein at least one of the two side parts (6, 7) is arranged to be displaceable or foldable on the base frame (2), such that lateral access to the seat area (3) of the treatment chair (1) is released, **characterised in that** handrails for the user to pull on, which are configured to measure an increase in physical strength due to magnetic stimulation, are arranged on the side parts (6, 7) or arm supports (8, 9), **in that** the magnetic coils (12, 13) are arranged to be displaceable in the longitudinal direction and/or transverse direction of the respective seat area (3) and/or backrest (4), and **in that** an operating panel (10, 11) with a display is configured to show graphically the position of the magnetic coils (12, 13) and the power increase via the handrails.

2. Treatment chair according to claim 1, **characterised in that** the side part (6, 7) can be displaced to the rear in the direction of the backrest (4) and thus laterally releases the seat area (3).

3. Treatment chair according to one of claims 1 or 2, **characterised in that** the side part (7) is arranged on the base frame (1) and is mounted to be displaceable along the base frame (2) in a guide rail (17).

4. Treatment chair according to claim 1, **characterised in that** the side part (6, 7) is arranged to be retractable on the base frame (1).

5. Treatment chair according to claim 1 or 4, **characterised in that** the side part (6, 7) can be pivoted to the rear about a vertical pivot axis (21) arranged on the rear part of the base frame (1) and thus laterally releases the seat area (3).

6. Treatment chair according to one of claims 1 to 5, **characterised in that** the side part (6, 7) has on its underside facing the floor at least one supporting roller (18) which rolls on the floor when displacing or pivoting the side part (6, 7).

7. Treatment chair according to one of claims 1 to 6, **characterised in that** it has a leg support (5) connecting to the seat area (3) and pivotable relative to the seat area (3).

8. Treatment chair according to claim 7, **characterised in that** a magnetic coil (14) is arranged in the region of the leg support (5).

9. Treatment chair according to one of claims 7 or 8, **characterised in that** the magnetic coil (14) is arranged to be displaceable in the longitudinal direction and/or transverse direction of the leg support (5).

10. Treatment chair according to one of claims 8 or 9, **characterised in that** the operating panel with the display is configured in order to show graphically the position of the magnetic coil (14).

11. Treatment chair according to one of claims 1 to 10, **characterised in that** it has measuring sensors to determine the body size of the user.

12. Treatment chair according to one of claims 1 to 11, **characterised in that** it is configured to carry out repetitive transpelvic magnetic stimulation TPM or repetitive peripheral magnetic stimulation rPMS or repetitive transcranial magnetic stimulation rTMS.

## Revendications

1. Fauteuil de traitement (1) pour la stimulation magnétique transpelvienne de tissu corporel, constitué d'un châssis de base (2) comprenant une assise (3) et un dossier (4), ainsi que de deux parties latérales (6, 7) comprenant des accoudoirs (8, 9), dans lequel dans l'assise (3) et/ou le dossier (4) est disposée au moins une bobine magnétique (12, 13), dans lequel l'une au moins des deux parties latérales (6, 7) est disposée de manière coulissante ou rabattable sur le châssis de base (2) de telle sorte qu'un accès latéral à l'assise (3) du fauteuil de traitement (1) soit libéré, **caractérisé en ce qu'**il est disposé sur les parties latérales (6, 7) ou les accoudoirs (8, 9) des poignées de maintien à tirer par l'utilisateur, qui sont formées pour mesurer une augmentation de la force musculaire en raison de la stimulation magnétique, que les bobines magnétiques (12, 13) sont disposées de manière coulissante dans la direction longitudinale et/ou la direction transversale de l'assise (3) et/ou du dossier (4) respectif, et qu'un panneau de commande (10, 11) comprenant un affichage est formé pour indiquer graphiquement la position des bobines magnétiques (12, 13) et l'augmentation de capacité par l'intermédiaire des poignées de maintien.

2. Fauteuil de traitement selon la revendication 1, **caractérisé en ce que** la partie latérale (6, 7) est coulissante vers l'arrière dans la direction du dossier (4) et libère ainsi latéralement l'assise (3).

3. Fauteuil de traitement selon l'une des revendications 1 ou 2, **caractérisé en ce que** la partie latérale (7) est disposée sur le châssis de base (1) et est supportée de manière coulissante dans un rail de guidage (17) le long du châssis de base (2).

4. Fauteuil de traitement selon la revendication 1, **caractérisé en ce que** la partie latérale (6, 7) est disposée de manière rabattable vers le bas sur le châssis de base (1).

5. Fauteuil de traitement selon la revendication 1 ou 4, **caractérisé en ce que** la partie latérale (6, 7) est pivotable vers l'arrière autour d'un axe de pivotement (21) vertical disposé sur la partie arrière du châssis de base (1) et libère ainsi latéralement l'assise (3).

6. Fauteuil de traitement selon l'une des revendications 1 à 5, **caractérisé en ce que** la partie latérale (6, 7) présente sur son côté inférieur tourné vers le sol au moins un galet de support (18) qui, lors du coulissement ou du pivotement de la partie latérale (6, 7), roule sur le sol.

7. Fauteuil de traitement selon l'une des revendications 1 à 6, **caractérisé en ce qu'**il présente un repose-jambes (5) raccordé à l'assise (3) et pivotable par rapport à l'assise (3).

8. Fauteuil de traitement selon la revendication 7, **caractérisé en ce qu'** une bobine magnétique (14) est disposée dans la zone du repose-jambes (5).

9. Fauteuil de traitement selon l'une des revendications 7 ou 8, **caractérisé en ce que** la bobine magnétique (14) est disposée de manière coulissante dans la direction longitudinale et/ou dans la direction transversale du repose-jambes (5).

10. Fauteuil de traitement selon l'une des revendications 8 ou 9, **caractérisé en ce que** le panneau de commande comprenant l'affichage est formé pour indiquer graphiquement la position de la bobine magnétique (14).

11. Fauteuil de traitement selon l'une des revendications 1 à 10, **caractérisé en ce qu'**il présente des capteurs de mesure pour déterminer la taille du corps de l'utilisateur.

12. Fauteuil de traitement selon l'une des revendications 1 à 11, **caractérisé en ce qu'**il est formé pour réaliser une stimulation magnétique transpelvienne répétitive TPM ou une stimulation magnétique périphérique répétitive rPMS ou une stimulation magnétique transcrânienne répétitive rTMS.
